# EUROPEAN PATENT SPECIFICATION

(11) **EP 2 001 409 B1**
(45) Date of publication and mention of the grant of the patent: **10.07.2019**
(21) Application number: 07753796.7
(22) Date of filing: 23.03.2007
(51) Int. Cl.: C12N 5/0775, A61F 2/28

(54) **FUNCTIONALIZED ARTIFICIAL BONE AND JOINT COMPOSITIONS AND METHODS OF USE AND MANUFACTURE**
FUNKTIONALISIERTE KÜNSTLICHE KNOCHEN- UND GELENKSZUSAMMENSETZUNGEN SOWIE VERFAHREN ZU IHRER HERSTELLUNG UND VERWENDUNG
COMPOSITIONS OSSEUSES ET ARTICULAIRES ARTIFICIELLES FONCTIONNALISÉES ET PROCÉDÉS POUR LES UTILISER ET POUR LES PRODUIRE

(30) Priority: 24.03.2006 US 785477 P
(43) Date of publication of application: 17.12.2008
(73) Proprietor: CAberTech, Inc., Harrison City, PA 15635 (US); Duquesne University of The Holy Spirit, Pittsburgh, PA 15282 (US)
(72) Inventor: McGowan, Kenneth, A., Harrison City, PA 15636 (US); Gawalt, Ellen, S., Pittsburgh, PA 15217 (US)
(74) Representative: FRKelly
(86) International application number: PCT/US2007/007197
(87) International publication number: WO 2007/111974

(56) References cited:
- WO-A1-2005/089826
- WO-A2-2004/072104
- WO-A2-2005/032417
- US-A- 4 668 295
- US-A- 5 241 094
- US-A1- 2003 215 484
- US-A1- 2004 265 571
- US-A1- 2005 049 717
- US-A1- 2006 194 008
- FISCHER H ET AL: "Bioactivation of inert alumina ceramics by hydroxylation", BIOMATERIALS, ELSEVIER SCIENCE PUBLISHERS BV., BARKING, GB, vol. 26, no. 31, 1 November 2005 (2005-11-01), pages 6151-6157, XP027767577, ISSN: 0142-9612 [retrieved on 2005-11-01]
- L.A. STERNBERGER ET AL.: 'A General Method for the Specific Purification of Antiprotein Antibodies' J. IMMUNOL. vol. 65, July 1950, pages 65 - 73, XP055194747
- GRAVES G A ET AL: "The Influence of Compisitional Variations on Bone Ingrowth of Implanted Porous Calcium Aluminate Ceramics", JOURNAL OF BIOMEDICAL MATERIALS RESEARCH, WILEY, NEW YORK, NY, US, vol. 9, no. 4, 31 July 1975 (1975-07-31), pages 17-22, XP002632647, ISSN: 0021-9304

## Description

### BACKGROUND OF THE INVENTION

### 1. Field of the Invention

The use of calcium aluminate (all associated phases, derivatives, and/or analogs thereof) as a raw material for the manufacture of artificial bone, artificial joints, in-vitro support structures, and support structure for tissue, cells, and/or organ growth and/or regeneration is provided. The use of slipcasting, slurrycasting or vibration casting in molds to generate the desired shapes of the artificial bones, joints and support structures of the invention is also provided. The present invention provides a functionalized composition comprising a calcium aluminate containing phase that is functionalized with a linking group comprising an organic acid molecule for providing a reactive location for the attachment of other chemical and biologic entities to the calcium aluminate containing phase.

### 2. Description of the Background Art

Current artificial joints and bones are manufactured from apatites or metal, typically titanium. They are machined to the desired shape which is a costly and production inefficient method of construction. These materials, in order to be accommodated by the host, must exhibit porosity so as to accommodate cell growth within the three dimensional structure. In particular, porosity is important where the part comes in contact with the host's natural structure (bone). This is due to the need for the host's bone to grow into and vascularize the artificial structure in order to develop the necessary bond between the two and reduce bone degeneration at the interface. Although attempts have been made in the current materials known by those skilled in the art to introduce porosity, the resulting structure is less than ideal. In most cases, artificial joints and other structures need to be replaced over time because the surrounding tissue and structure has degenerated. Pins, screws, rods and other structures are required to stabilize, bond and support the interface.

There is an identifiable need to create structures designed to support tissue growth, such as in artificial organ growth. The use of plastics as a support structure for tissue growth is known by those skilled in the art and has been accomplished by the use of organic polymers. These plastics and polymers are expensive when employed as artificial prostheses and lack porosity.

Bone is a very complex organ made of cells and extracellular matrix. It is constantly being rebuilt through the interactions of osteoblasts and osteoclasts. Bone functions include maintaining blood calcium levels, providing mechanical support to soft tissues, and serving as levers for muscle action, supporting haematopoiesis, and housing the brain and spinal cord.

The treatment of bone diseases and fractures represents one of the largest markets for regenerative medicine, estimated to reach $1.8 billion by 2008. Annually, there are over 500,000 bone graft procedures in the United States. Of these procedures, only 10% involve synthetic sources.

Bone fracture and damage result in more than 1.3 million surgical procedures each year only in the United States. Autografts and allografts are considered the standard for treating these types of wounds. However, both methods have disadvantages. The failure rate of an autograft is controlled by the need of a second site of surgery. The necessity of a second site of surgery for donor material contributes to the failure rate of autograft procedures. Failure can be attributed to limited supply, inadequate size and shape, and the morbidity associated with the donor site; all of which are issues of concern. Allografts share some of these disadvantages, and in addition; the procedure raises questions associated with donor and recipient compatibility. The disadvantages of autografts and allografts have influenced the importance of synthetic bone implants. The calcium aluminate materials of the present invention are effective bone replacement material. It will be appreciated by those skilled in the art that the calcium aluminate materials of the present invention have a controlled porosity, high strength and ease of casting, and overcome many of the difficulties associated with currently available technology.

Current implant technologies involve the use of titanium and other metals which is costly due to the need to machine the material to the desired shape. These materials, in order to be accommodated by the host, must exhibit porosity where the implant comes in contact with the host's bone. This is due to the need for the host's bone to grow into and vascularize the artificial structure in order to develop the necessary bone between the two, and reduce bone degeneration at the interface. In addition, there is bone loss due to the hardness differential between the implanted metal and natural bone. Plastics are often inserted between the two to stop this from occurring but this affects the ability of the appropriate interface to form. Metal implants require the use of rods, pins, and screws to be held into place and often need to be replaced over time.

As stated herein, methods to heal damaged bone include autografts, allografts, and the use of synthetic sources. Autografts are the standard for repairing skeletal defects, however, there are disadvantages associated with this type of treatment. Reasons for failed autografts include the need for a second site of surgery, limited supply, inadequate graft size and shape, and morbidity associated with the donor site. Allografts present similar disadvantages and are further complicated by issues related to the potential of pathogenic transmission.

Synthetic sources and bone substititutes are being evaluated to overcome the difficulties involved with autografts and allografts. To be effective in healing bone defects a material needs to have certain qualities. A scaffold should be porous to allow nutrients to permeate, and permit vascularization. It should be osteoconductive to enable new bone tissue formation, and it should degrade to allow resorbtion. Demineralized bone matrix, hydroxyapatite and tricalcium phosphate granules and scaffolds, organic sponges, synthetic sponges, porous ceramics, and collagen discs have all been used as bone substitutes or vehicles to deliver bone cells or growth factors.

Current artificial joints and bones are manufactured from apatites or metal, typically titanium. They are machined to the desired shape which is a costly and production inefficient method of construction. These types of materials are not all optimized for porosity, which is crucial for a successful implant material. There is a need for the host's bone to grow into and vascularize the artificial structure in order to develop the necessary bond between the artificial and natural bone matrix. This will result in reduced bone degeneration at the interface of the implant material and natural bone.

Research to date has mainly focused on calcium aluminates for use in the dental industry as a direct restorative dental material wherein the calcium aluminate cement is used as a fine bonding agent in the matrix and is not the primary support aggregate.

Calcium aluminates do not cause an inflammatory response when placed into *in-vivo* scenarios. Klawitter and Hulbert studied the influence of pore structures on calcium aluminate pellets. Calcium aluminates were implanted into the midshaft region of dog femurs and showed no inflammatory response. The Klawitter and Hulbert study showed that tissue around the porous implants healed more quickly.

Other studies have shown that calcium aluminates are able to support the proliferation of cells. Kalita studied the porosity of calcium aluminates and determined that some of the pores of the calcium aluminates were almost filled with a monolayer of cells. Kalita used fused deposition process to build materials using a computer program which constructs the material layer by layer.

US2005/049717A1 teaches a ceramic porous body for in-vitro and in-vivo use comprising a composition comprising a calcium aluminate (CA) containing phase

WO2005/089826A1 is concerned with providing bovine, porcine, and autogenous bone graft materials and titanium implants that may be modified with active peptides for repair and regeneration of periodontal bone

WO2004/072104A2 teaches nanocrystalline surface coatings upon titanium surfaces and attachment of peptide amphiphile nanofibers

WO2005/032417A2 teaches antimicrobial hyaluronic acid coatings upon an implant that is metal, metal alloy, or a calcium phosphate ceramic

Fischer et al (2005) Biomaterials (Elsevier) vol. 26 no. 31 pages 6151-6157 teaches treatment of high purity alumina (Al₂O₃) with sodium hydroxide to bioactivate the surface of the alumina

Graves and Villanueva (1975) J. Biomed. Mater. Res Symposium No. 6, pp17-22 reports the influence of compositional variations of phosphorous pentoxide on bone ingrowth of implanted porous calcium aluminate ceramics.

US4668295 describes the use of a polyfunctional carboxylic acid group that is intimately mixed with, and chemically consumed in, a reaction with the bone substitute when water is added to the composition mixture in order to effect the production of and set hardening of the composition

In spite of this background art, there remains a very real and substantial need for ceramic porous bodies comprising calcium aluminate, its phases, derivatives and/or analogs thereof, wherein the ceramic bodies are capable of functioning as artificial bone, artificial joints, in-vitro support structures, and in-vivo support structures for cells, tissues, organs and nerve growth and regeneration.

### SUMMARY OF THE INVENTION

The present invention has met the above-described need. The present invention provides a functionalized ceramic body(ies) and functionalized compositions comprising a calcium aluminate containing phase. The calcium aluminate containing compositions of this invention may be used in the manufacture of artificial prostheses that may function as artificial bones and joints of a patient, as well as in-vitro support structures, in-vivo support structures for cells, tissues, organ and nerve growth and/or regeneration. The present invention provides a functionalized ceramic body for in vitro and in vivo use comprising a calcium aluminate containing phase that is functionalized with a linker group (or linking group or coupling agent) comprising an organic acid molecule for providing a reactive location for the attachment of at least one of another chemical moiety (molecule) or a biologically active moiety to the ceramic body wherein said organic acid molecule has a formula of (CO₂H)ₐCₙH_{2n/2n-2}X_{d}, wherein X is selected from the group of CH₃, COOH, N, N-dicyclohexylcarbodiimide (DCC)-coupled COOH, OH, NH₂, a phosphate moiety and Br, wherein a is a number from 1 to 3, wherein n is a number from 12 to 18, and wherein d is a number from 1 to 3, said reactive location being subsequently reacted with said other chemical moiety or said biologically active moiety to attach said other chemical moiety or said biologically active moiety to said functionalized calcium aluminate containing phase.

. The functionalized ceramic body includes wherein the calcium aluminate containing phase comprising one or more phases, analogs and derivatives of calcium aluminate that result from the interaction of CₙA_{y}, and CₙA_{y}-hydrates, wherein n is about 12, y is an integer from about 1 to about 24, C is CaO, and A is Al₂O₃. In a preferred embodiment, the functionalized ceramic body includes wherein the organic acid has a formula of (CO₂H)ₐCₙH_{2n/2n-2} X_{d}, wherein X is selected from the group of CH₃, COOH, DCC-coupled COOH, OH, NH₂, and Br and wherein a is a number from about 1 to about 3, wherein n is a number from about 12 to about 18, and wherein d is a number from about 1 to about 3. In a more preferred embodiment, the functionalized ceramic body includes wherein an antibiotic is the moiety attached to the calcium aluminate containing phase.

Optionally, the functionalized ceramic body further comprises at least one of a retarder, water, an accelerator, a surfactant, a foaming agent, a fiber, a source of phosphate, an orthophosphate, and a reactive alumina, and combinations thereof.

A more preferred embodiment of this invention provides wherein the functionalized ceramic body as described herein is functionalized with the organic acid and subsequently reacted with 3-maleimidopropionic acid N-hydroxysuccinimide ester (NHS) for binding one or more peptides to an amino-functionalized calcium aluminate containing phase.

The functionalized ceramic body may be an artificial prosthesis wherein the artificial prosthesis is selected from the group consisting of an artificial bone, artificial joint, in-vitro support structure, an in-vivo support structure, and a scaffolding matrix for support of cell, tissue, organ, and nerve growth.

This invention also provides a functionalized composition comprising a calcium aluminate containing phase that is functionalized with a linker (also referred to herein as a linker group, linking group, modifier, or coupling group) comprising an organic acid molecule for providing a reactive location for the attachment of at least one of another chemical moiety (molecule or compound) or a biologically active moiety to the calcium aluminate containing phase, wherein said organic acid molecule has a formula of (CO₂H)ₐC₂ₙH_{2n/2n-2}X_{d}, wherein X is selected from the group of CH₃, COOH, DCC-coupled COOH, OH, NH₂, a phosphate moiety and Br, wherein a is a number from 1 to 3, wherein n is a number from 12 to 18, and wherein d is a number from 1 to 3, said reactive location being subsequently reacted with said other chemical moiety or said biologically active moiety to attach the other chemical entity or the biologically active moiety to said functionalized calcium aluminate containing phase.

The functionalized composition comprises a calcium aluminate containing phase comprising one or more phases, analogs and derivatives of calcium aluminate that result from the interaction of CnAy and CnAy-hydrates, wherein n is about 12, y is an integer from about 1 to about 24, X is a phosphate moiety, C is CaO, and A is Al₂O₃.

In a preferred embodiment of this invention, the functionalized composition as described herein includes wherein an antibiotic is the moiety attached to the calcium aluminate containing phase. The antibiotic may be any antibiotic, such as for example but not limited to ampicillin or vancomycin.

Optionally, the functionalized composition as described herein comprises at least one of a retarder, water, an accelerator, a surfactant, a foaming agent, a fiber, a source of phosphate, an orthophosphate, and a reactive alumina, and combinations thereof.

In a more preferred embodiment of this invention, the functionalized composition as described herein comprises a calcium aluminate containing phase that is functionalized with the organic acid and subsequently reacted with 3-maleimidopropionic acid N-hydroxysuccinimide ester (NHS). This is useful, for example but not limited to, for the binding one or more peptides to the amino-functionalized calcium aluminate containing phase.

This invention also provides for a method of making an artificial prosthesis comprising the steps of mapping of a patient's identified bone or tissue structure; creating a three dimensional pattern of the identified bone or tissue structure from the mapped bone or tissue structure; creating a mold or negative of the identified bone or tissue structure from the pattern; casting a functionalized composition (as described herein) comprising a calcium aluminate containing phase (as described herein) that is functionalized with a linker group comprising an organic acid molecule for providing a reactive location for the attachment of at least one of another chemical moiety (molecule or compound) or a biologically active moiety to the calcium aluminate containing phase, into the mold thereby forming the artificial prosthesis. In a preferred embodiment, the method as described herein includes wherein an antibiotic is the moiety attached to the calcium aluminate containing phase. The method also includes wherein the functionalized composition further optionally comprises at least one of a retarder, water, an accelerator, a surfactant, a foaming agent, a fiber, a source of phosphate, an orthophosphate, and a reactive alumina, and combinations thereof. In a preferred embodiment of this method, as described herein, the calcium aluminate containing phase is functionalized with the organic acid and subsequently reacted with 3-maleimidopropionic acid N-hydroxysuccinimide ester for binding one or more peptides to an amino-functionalized calcium aluminate composition. The functionalized compositions may be any of the compositions of this invention as described herein.

The present invention provides a ceramic body for in vitro and in vivo use comprising the compositions of this invention as described herein. The ceramic body of this invention has a porosity suitable for achieving vascularity.

The functionalized ceramic bodies, functionalized compositions, and methods of this invention will be more fully understood from the following descriptions of the invention and the claims appended hereto.

### BRIEF DESCRIPTION OF THE DRAWINGS

Figure 1. MG-63 cells attach and proliferate on compositions comprising a calcium aluminate containing phase of the present inventions under static (A) and dynamic (B) culture conditions as assessed by CyQuant assay.
Figure 2. MG-63 cells maintain high viability when seeded and cultured on compositions comprising a calcium aluminate containing phase of the present invention.
Figure 3. MG-63 cells attach on compositions comprising a calcium aluminate containing phase of the present invention as assessed by scanning electron microscopy.
Figure 4. Human adult mesenchymal stem cells attach and show high viability but variable proliferation on compositions comprising a calcium aluminate containing phase of the present invention under static (A) and dynamic (B) culture conditions.
Figure 5. Human adult mesenchymal stem cells attach on compositions comprising a calcium aluminate containing phase of the present invention as assessed by scanning electron microscopy.
Figure 6. hAMSC on CA express alkaline phosphatase in response to an osteogenic supplement.
Figure 7. Compositions comprising a calcium aluminate containing phase of the present invention is non-toxic as assessed by chick CAM assays.

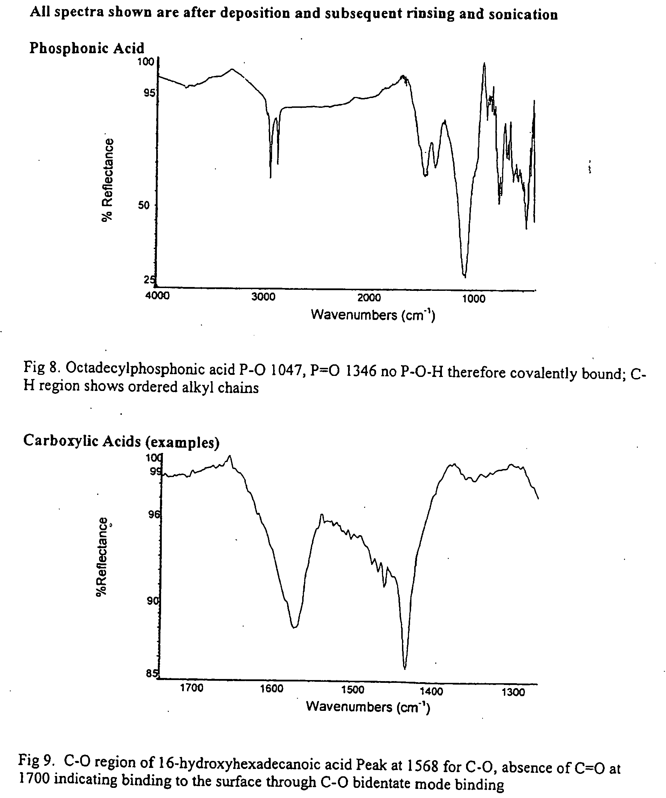

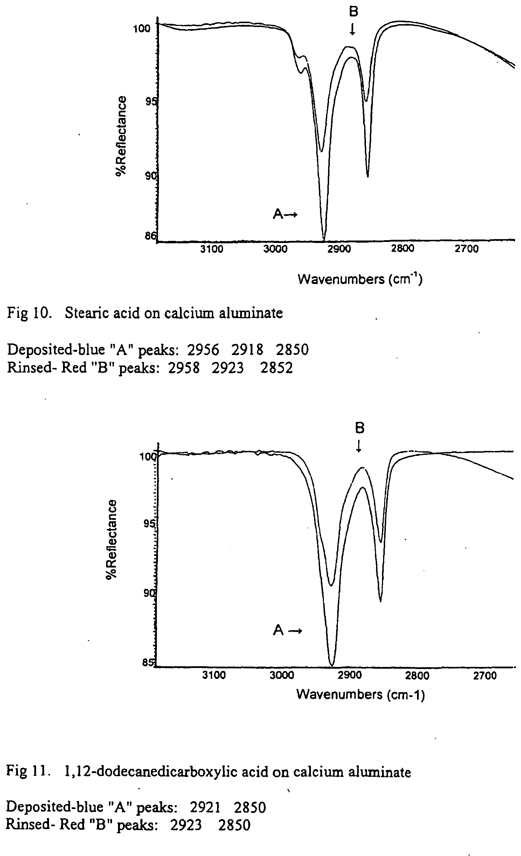

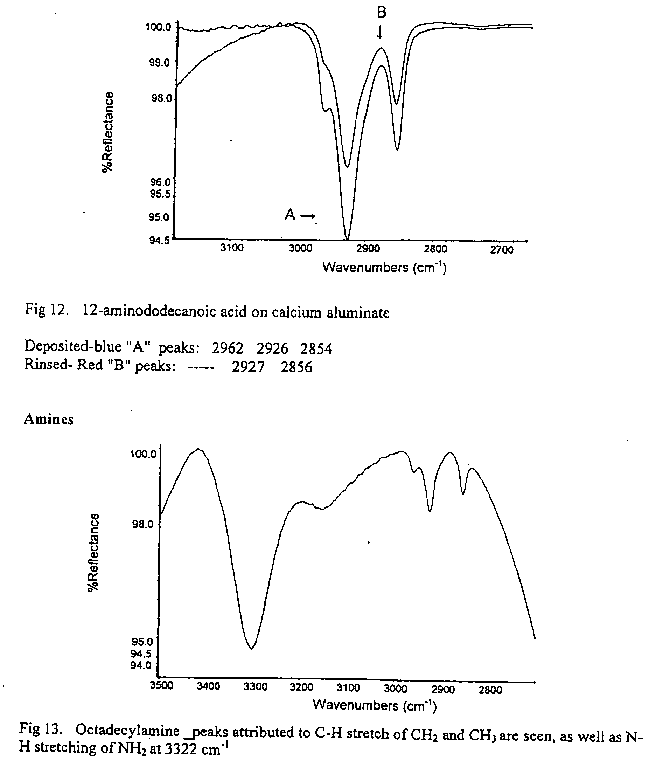

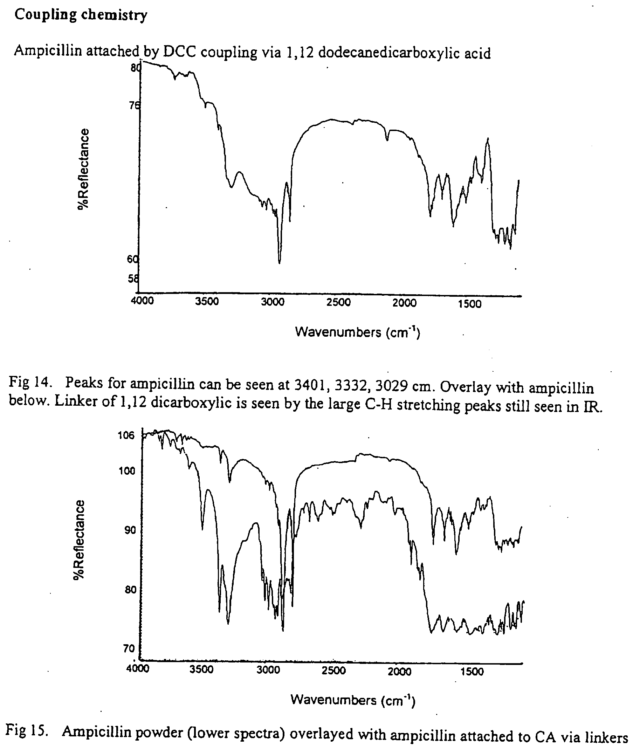

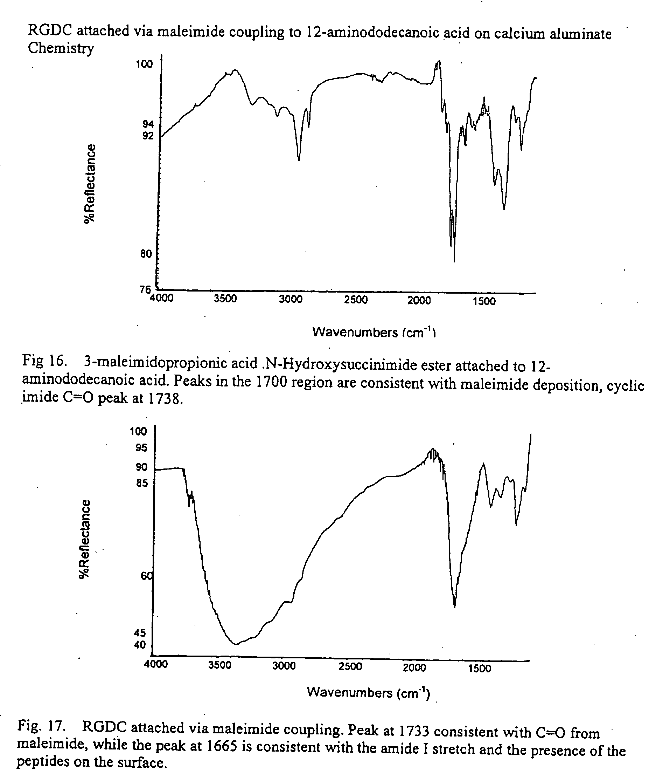

Figures 8-17 Show spectra of various embodiments of the functionalized compositions comprising a calcium aluminate containing phase of the present invention.

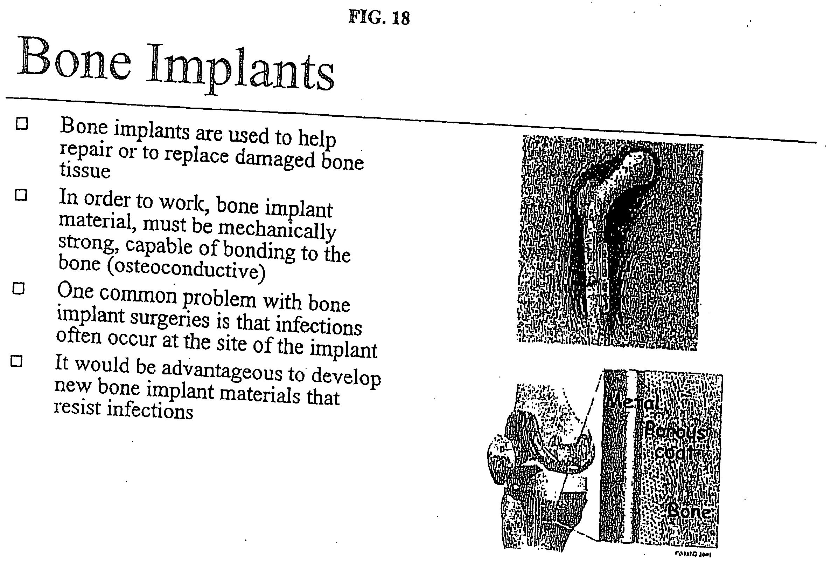

Figures 18 Shows bone implants used to help repair or replace damaged bone.
Figure 19 Shows a preferable form of the compositions comprising a calcium aluminate containing phase of the present invention and the functionalized "interface" between the "surface" of the compositions and the biologic "tissue".
Figure 20 shows that by varying the functionality of the head and tail groups of acids used as linkers that the calcium aluminate containing compositions may be modified in many ways to provide reactive locations for attachment of other chemical moieties and/or biologic moieties.
Figure 21 shows phosphonic acid as the linker to functionalize the calcium aluminate containing phase of the compositions .
Figure 22 shows an example of carboxylic acid binding onto the surface of a calcium aluminate containing composition of this invention. The spectra shown is that of stearic acid absorbed on the surface of a calcium aluminate containing composition.
Figure 23 shows the functionalization of a calcium aluminate containing composition of this invention with organic acid tail group variations.
Figure 24 shows the functionalization (coupling) for bonding an antibiotic, namely ampicillin, to a calcium aluminate containing composition of this invention.
Figure 25 shows the spectra of an antibiotic, namely ampicillin, linked to a dicarboxylic-functionalized calcium aluminate containing composition of this invention.
Figure 26 shows RGDC peptide coupling to an amino-functionalized calcium aluminate containing composition of this invention.

### DETAILED DESCRIPTION OF THE INVENTION

Calcium aluminate and its representative phases, analogs, and derivatives (including such as for example the introduction of phases resulting from the interaction of CₙA_{y}, and Cₙ A_{y}-Hydrates, are better alternatives as an artificial material, wherein preferably, n is about 12 and y is an integer from about 1 to 24. There are several reasons for this including the fact that CₙA_{y} contains hydratable compounds that introduce needed strength into the matrix via a dissolution/precipitation reaction. Porosity is easily introduced into the structure via the aggregate itself and/or through the use of a foaming agent. The resulting matrix can be cast to a specific shape with ease using casting technology known by those persons skilled in the art, such as including, slip casting, slurry casting or vibration casting into molds to generate a desired shape. The resulting calcium aluminate containing composition is chemically compatible with bone and other biological processes. The resulting shape having the calcium aluminate containing composition can be high fired to make it unreactive with its environment, if desired, or, it can be partially fired to leave it somewhat active, or it may be unfired. Furthermore, a hollow cavity within the structure can be created to better allow vascularity to occur and to allow marrow to exist if indeed the body will begin to produce it with the presence of vascularity. Both conditions of vascularity and marrow growth will foster the progress of each process.

The present invention provides an artificial prosthesis having the CₙA_{y} material as a structure to support tissue growth. It can be pre-engineered to match the desired finished structure and in addition, in the form of hydrates, these materials will slowly be metabolized by the body. Because of the nature of the compounds, they can easily be derivatized and functionalized for use with biological processes, such as for example, but not limited to, accommodating protein building blocks, and binding sites.

The present invention provides artificial prosthesis structures and a method for the manufacture of an artificial prosthesis. The method includes mapping the structure of interest by digitizing data from MRI scans (if soft tissue), X-ray data (if bone structure) or a combination of both, digitizing the data to create a three dimensional pattern or blank of the structure as known by those skilled in the art, utilizing the blank or pattern to create a mold or negative of the structure of interest, casting within the mold the compositions of the present invention comprising the CₙA_{y}, and CₙA_{y} hydrates of this invention, and optionally adding biologically active materials to produce an artificial prosthesis. Optionally, the resulting artificial prosthesis is then further processed, if desired. This may involve a firing process to fix certain desired mineralogical phases and/or chemically activated by an immersion process known by those persons skilled in the art.

The present invention provides a ceramic body for in vitro and in vivo use comprising a calcium aluminate containing phase. In preferred embodiments of this invention the composition comprising the calcium aluminate containing phase further comprises one of a foaming agent, a fiber, a source of phosphate, an accelerator, a retarder, a surfactant, reactive alumina, a biologically active material, and combinations thereof.

In another embodiment of this invention the ceramic body as described includes wherein the calcium aluminate containing phase comprises one or more phases, analogs and derivatives of calcium aluminate.

In a preferred embodiment of this invention, the ceramic body as described herein includes wherein the calcium aluminate containing phase results from the interaction of CₙA_{y}, and CₙA_{y}-hydrates, wherein n is an integer about about 12, y is an integer from about 1 to about 24.

In another embodiment of this invention, a composition is provided that comprises a calcium aluminate containing phase and optionally at least one or more of a retarder, and a surfactant, wherein the calcium aluminate containing phase results from the interaction of CₙA_{y} and CₙA_{y}-hydrates, wherein n is an integer from about 12, y is an integer from about 1 to about 24. The composition optionally further comprises at least one of a fiber, water, an accelerator, a biologically active material, a source of phosphate, a reactive alumina, and combinations thereof.

In yet another embodiment of this invention, a method is provided for using a composition for producing artificial structures for use in-vitro or in-vivo by a patient comprising employing a composition comprising a calcium aluminate containing phase, and optionally at least one or more of a retarder, and a surfactant, as described herein, placing the composition into a mold wherein said mold matches a patient's pre-identified structure to form an artificial structure. The method includes wherein said calcium aluminate containing phase results from the interaction of CₙA_{y} and CₙA_{y}-hydrates, C wherein n is an integer from about 12, y is an integer from about 1 to about 24. The present composition may also be used as an in-situ patch for repairing a bone void of the patient that may occur, for example but not limited to, as a result of trauma and injury to the bone. The composition may be fired or unfired.

The present invention includes the calcium aluminate biomaterials as described herein that have been modified by, such as for example, an organic acid to enable the attachment of other moieties thereto such as for example antibiotics, peptides or proteins. More specifically, the modified calcium aluminate biomaterials of the present invention comprise the ceramic body as described herein and human adult mesenchymal stem cells (hAMSC) and/or osteo-blast like MG-63 cells that are attached to the ceramic body that has been modified by the organic acid.

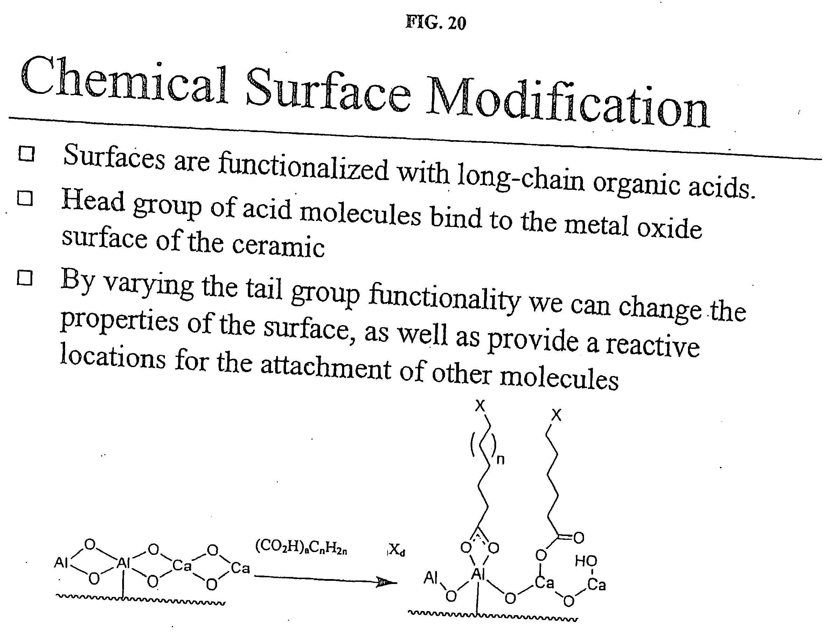

The modification of the ceramic body or the compositions comprising a calcium aluminate containing phase of the present invention by organic acids enhances the ability of the ceramic body or the compositions to act as a bone stabilizer. Organic acids were used as the modifiers (linkers) and were found to bind irreversibly to the ceramic body and compositions set forth herein. The organic acid modifiers (linkers) employed in the functionalization of the ceramic body and compositions of this invention are molecules comprising a head group with a pKa less than about 15, such as for example but not limited to phosphonic acid, carboxylic acid, and amines, followed by an alkyl chain, and a variable tail group. The functionality of the tail group may be varied so that the organic acid modifier may act as a linker between the ceramic body or compositions of the present invention, and another organic or biologic molecules of choice. The organic acid modifier is bound to the ceramic body or compositions of the present invention and can not be removed by chemical or mechanical agitation. The attachment is preferably done post-production of the ceramic body. With regard to the composition(s) comprising the calcium aluminate containing phase(s) of this invention, the attachment of the linking group may be performed prior to or post formation of the three dimensional shape. Figure 20 shows the reaction between the compositions comprising a calcium aluminate containing phase, wherein the organic acid linking group has the formula (CO₂)ₐCₙH_{2n/2n-2} X_{d}, wherein X is selected from the group of CH₃, COOH, DCC-coupled COOH, OH, NH₂, and Br and wherein a is a number from about 1 to about 3, wherein n is a number from about 12 to about 18, and wherein d is a number from about 1 to about 3, and wherein X is the variable tail group, and the resulting attachment of the organic linker to the compositions of this invention. The organic functionalization of the compositions comprising a calcium aluminate containing phase adds chemical variabilility to the modified compositions by presenting chemically reactive groups at the interface of the pores and surface with the environment in which the modified composition is located.

The tail groups "X" as described herein may be used for several purposes. For example, the variation of the tail group may be used to control and tailor the ceramic body's interfacial properties such as for example but not limited to hydrophobicity. The tail group may also be reacted further with other organic molecules to tether small molecules or macromolecules to the ceramic body or compositions of the present invention in order to control the ceramic body's or composition's properties and function. Further, the tether may serve a delivery function by allowing for the deposition of desired organics to a specific location, especially in biologic applications. These molecules may include for example, but are not limited to, polymers, peptide sequences, proteins, antibiotics, and blood thinning pharmaceuticals.

The modified ceramic body and compositions of the present invention may be used as bone stabilization materials. The organic molecule functionalization of the ceramic body and compositions as described herein brings added value due to the flexibility of the method which allows for the attachment of many different types of molecules that may be useful in a biological setting.

The modified calcium aluminate compositions of the present invention may be useful in the industrial setting for example in cements. The organic molecule functionalization of the calcium aluminate of the present invention adds chemical functionality and variability to the cement which did not exist before. The functionalization of the calcium aluminate cement surface is useful for example for adhesive or protective purposes.

The modified ceramic body and modified calcium aluminate compositions of the present invention may be hydratable or nonhydratable, and may be useful as a bone void filler, a bone graft extender, a three dimensional resorbable tissue scaffold, and as an implant such as for example a long bone replacement and/or repair. The modified ceramic body and modified calcium aluminate containing compositions of the present invention have biocompatibility as demonstrated by human adult mesenchymal stem cells, embryonic chick chorioallantoic membrane assay, and rat calvarial defect animal testing. The ease of casting and the strength and porosity of the ceramic body of the present invention are advantages over current bone graft technologies and will be useful in combat support and military medical centers. It will be appreciated that the calcium aluminate containing compostions of the present invention may be manufactured having different synthetic conditions with varying porosities, hydratability and surface roughness, for example.

Calcium aluminate compositions of the present invention with controlled porosity and high strength show potential as bone replacement materials. Our results indicate that calcium aluminate containing compositions of the present invention are biocompatible with human MG-63 osteoblast-like cells and human adult mesenchymal stem cells in both dynamic and static *in vitro* culture conditions.

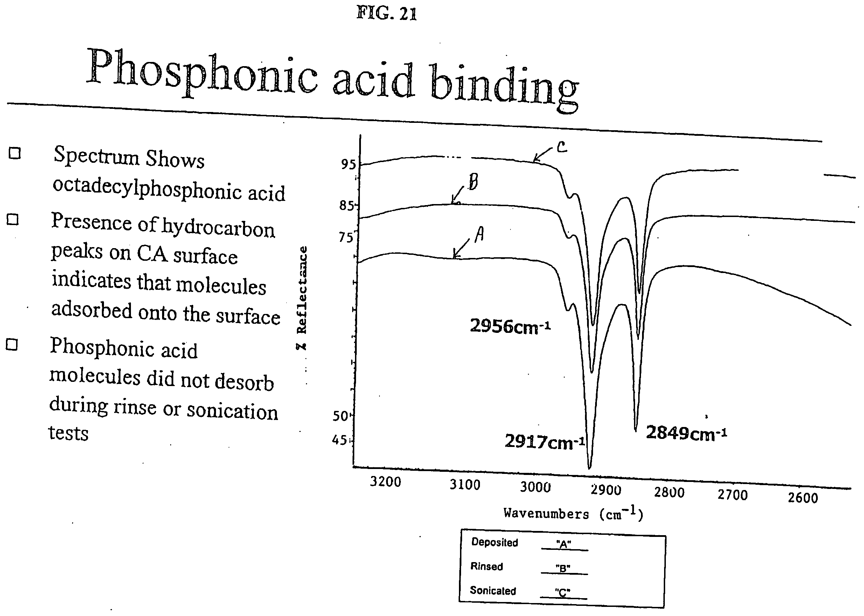

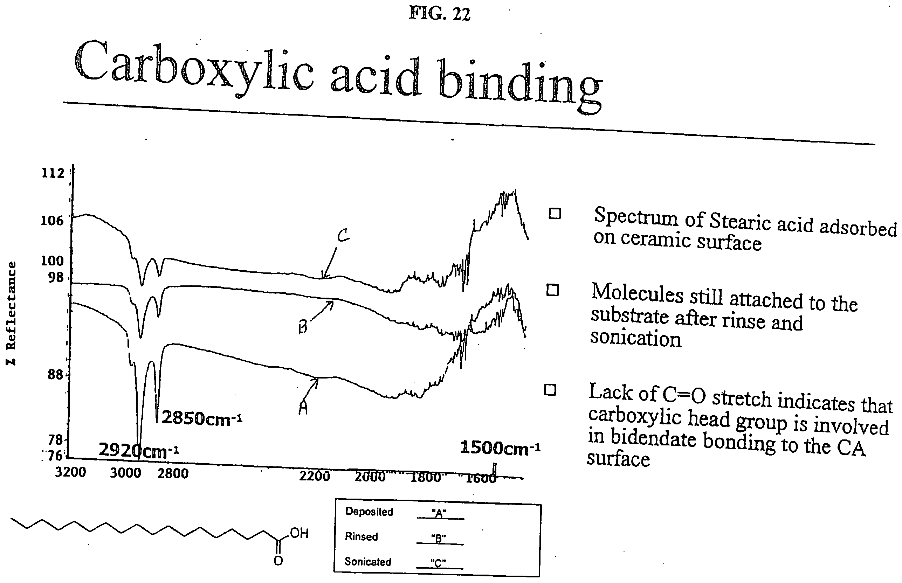

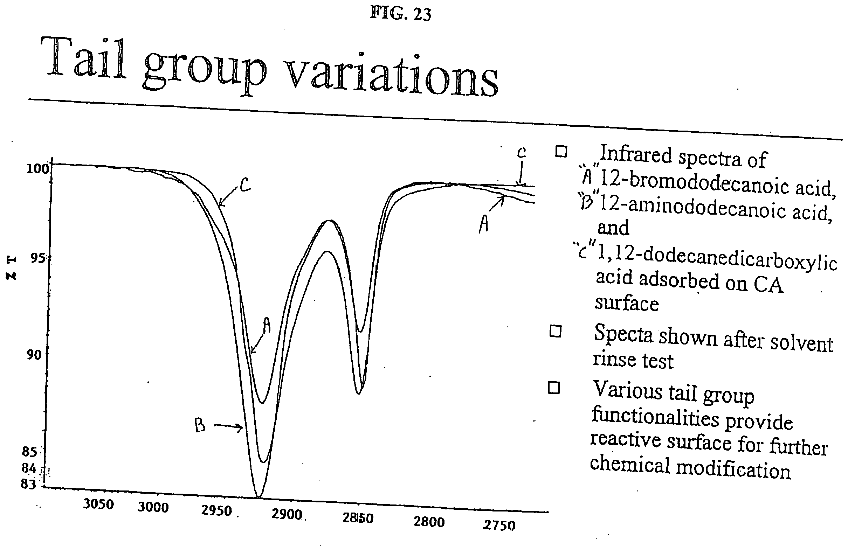

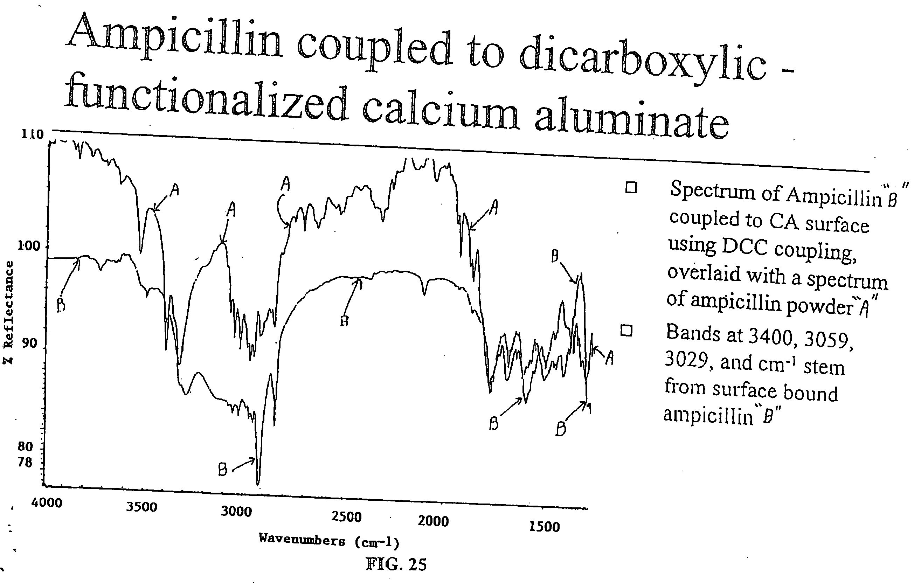

Figures 20-26 show examples of molecules that may be attached to the ceramic body or the calcium aluminate containing compositions or calcium aluminate materials of the present invention. As used herein, these molecules are referred to as a linker(s), a linker group, a linking group, a modifier, or as a coupling agent, and are an organic acid molecule. For example, but not limited to, the following molecules are a linker, a linking group, or a coupling agent that may be attached to the ceramic body or the calcium aluminate containing compositions of this invention: Octadecylcarboxylic acid, 12-romododecanoic acid, 1,12-dodecanodicarboxylic acid, and 16-hydroxyhexadecanoic acid. The reaction scheme set forth immediately below shows one embodiment of the modication of the calcium aluminate material of the present invention. 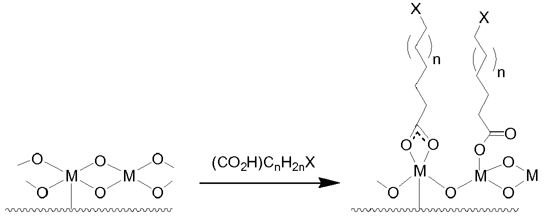 Reaction between ceramic and organic, where M= A1 or Ca and X is the variable tail group.

The following shows the infrared spectra of the C-H region of a calcium aluminate material of the present invention functionalized with octadecylcarboxylic acid. 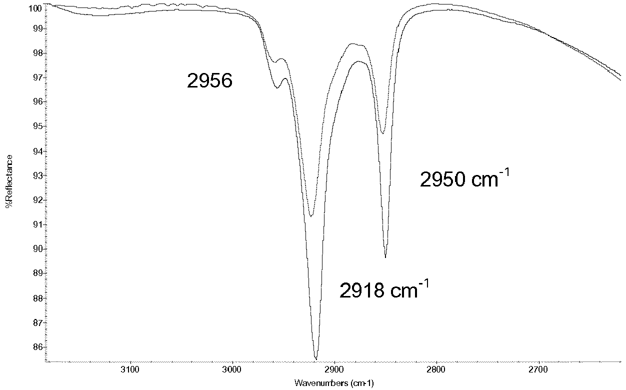 The above graph forth the infrared spectra of the C-H region for a calcium aluminate material of the present invention modified with octadecylcarboxylic acid attached to it through the carboxylic acid head group. The peaks are due to C-H stretching of the CH₂ groups in the alkyl chain and the CH₃ terminus (2956 cm⁻¹). The blue spectra, labeled "A", is before rinsing and the red spectra, labeled "B", is after rinsing.

Examples of organic molecules that may be tethered to the surface of the calcium aluminate materials of the present invention include 3-maleimidopropionic acid N-hydroxysuccinimide ester, Dicyclohexylcarbodiimide, Arginine-Glycine-Aspartic Acid-Cysteine attached via 3 maleimido---to 12-aminododecanoic acid (see figure immediately below), and Ampicillin via dicyclohexylcarbodiimide coupling on 1,12 dodecanedicarboxylic acid. 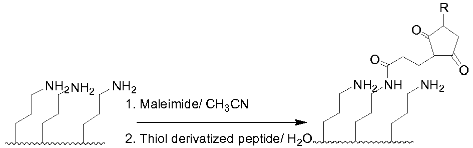 The above reaction scheme shows maleimide coupling of cysteine derivatized peptides to the surface of calcium aluminate material of the present invention.

All molecules were initially adhered to the surface of the calcium aluminate materials of the present invention using a 1 hr dip in a ImM - lOmM solution of organic and THF (tetrahydrofuran), followed by 30 minutes to about 1hour in a 120 degrees C (Centigrade) oven. Rinsing consists of rinsing the solvents in THF and then sonicating them for 30 minutes in THF.

The maleimide coupling procedure is set forth in the reaction set forth immediately above and is followed by evacuation of solvent at 0.1Torr. This coupling procedure, for example, may be employed for thiol or cysteine derivatized molecules such as peptides and proteins.

The DCC coupling procedure, known by those skilled in the art, may also be employed to link carboxylic acid terminated molecules to molecules with a reactive -OH group.

The present invention employs coupling procedures to attach molecules to the ceramic body or compositions comprising a calcium aluminate containing phase of this invention via a OH, NH2, COOH, or Br reactive tail group. It will be appreciated by those persons skilled in the art that this procedure allows access to SN2 chemistry, amide coupling, and Michael additions (via maleimide), for example.

Those persons skilled in the art will appreciate that the persistence of the CH₂ groups in the IR spectra confirms that the alkyl chain is still present on the surface of the modified calcium aluminate materials of the present invention after chemical and mechanical agitation.

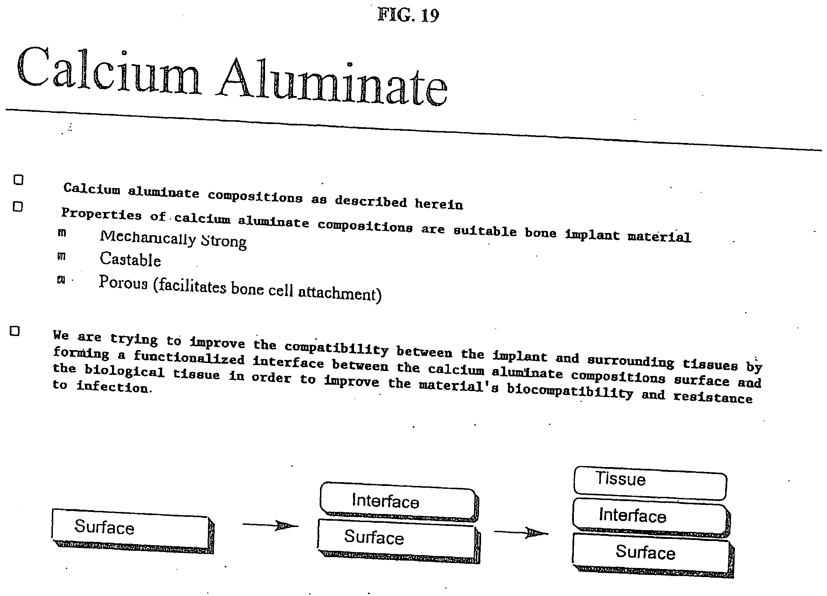

We have tested MG-63 (osteoblast-like cells) and human adult mesenchymal stem cells without inducing them into osteoblasts and inducing them into osteoblasts through serum additives. It will be appreciated that the modified porous ceramic body and the modified calcium aluminate materials of the present invention improve their effectiveness as biomaterials. The adhesion and bonding of various molecules, for example, but not limited to, long chain alkanes, with different head and tail functional groups to the surface of ceramics were tested and the results set forth herein and in the attached figures (see especially Figures 19-26). The surfaces of the modified calcium aluminate materials of this invention were analyzed with diffuse reflectance infrared fourier transform spectroscopy (DRIFT). Additionally, the surface of the modified calcium aluminate material of this invention has been functionalized with antibiotic molecules.

Attachment, proliferation, and viability of MG-63 cells and hAMSC were assessed using the CyQuant assay, scanning electron microscopy, and fluorescent viability stains. The viability of cells when attached to the ceramic body of the present invention is above 90%, and cells proliferate when attached to the calcium aluminate surface.

Over the course of 14 days in an osteogenic supplement, differentiation is visible as indicated by increased alkaline phosphatase activity, a marker for osteoblast differentiation. *In vivo* chick chorioallantoic membrane (CAM) assays also indicate the biocompatibility of calcium aluminate. This suggests that the functionalized compositions, functionalized prosthesis, and/or the functionalized ceramic bodies of the present invention may be implanted into rat calvarial defects. Based on the *in vitro* and preliminary *in vivo* studies, calcium aluminate-based materials of the present invention are an effective material for bone regenerative medicine. Calcium aluminates of the present invention are biocompatible and can be used in bone tissue engineering, when seeded with human adult mesenchymal stem cells differentiated into osteoblasts. This study focuses on the use of the calcium aluminate materials of the present invention as a bone substitute, however, as a scaffolding material, the calcium aluminate materials of the present invention may be used to support a variety of other cell types including such as for example but not limited to nerve and organ tissues.

The calcium aluminate materials of the present invention may be employed as a bone replacement material. The calcium aluminate materials of the present invention having controlled porosity, and high strength, and their ease of forming complex three-dimensional shapes, overcome many of the difficulties associated with the background technology. Calcium aluminate morsels comprising the calcium aluminate material of the present invention were chosen as the material for use as a bone void filler, as set forth in the examples.

We have developed a novel method to produce the calcium aluminates of the present invention that is inexpensive, relatively easy, and economical. In our preparation of the calcium aluminates of the present invention, we are able to control phase composition, microporosity, surface texture and dissolution rate. Through our novel approach we are synthesizing compositions comprising a calcium aluminate containing phase(s) with defined and controlled macro-porosity and volume stability.

The cellular assessment of calcium aluminate materials for bone tissue engineering is set forth herein. We have studied the biocompatibility of calcium aluminates through the attachment and proliferation of MG-63 cells and human adult mesenchymal stem cells on the calcium aluminate materials of the present invention. MG-63 cells are an osteoblast like cell line. Bone marrow-derived human adult mesenchymal stem cells (hAMSC) are a population of multi-potent cells with the ability to differentiate into osteogenic cells. Given that hAMSC can be directed into the osteogenic lineage *in vitro* in the presence of a dexamethasone-containing osteogenic supplement, we have also evaluated osteoblastic differentiation of hAMSC on the calcium aluminate material of the present invention.

In another embodiment of this invention a functionalized ceramic body for in vitro and in vivo use comprising a calcium aluminate containing phase that is functionalized with a linker group (or linking group or coupling agent) comprising an organic acid molecule for providing a reactive location for the attachment of at least one of another chemical moiety (molecule) or a biologically active moiety to the ceramic body. The functionalized ceramic body includes wherein the calcium aluminate containing phase comprising one or more phases, analogs and derivatives of calcium aluminate that result from the interaction of CnAy and CnAy-hydrates, wherein n is an integer from about 12, y is an integer from about 1 to about 24, X is a phosphate moiety, C is CaO, and A is Al₂O₃. The functionalized ceramic body includes wherein the organic acid has a formula of (CO₂H)ₐCₙH_{2n/2n-2} X_{d}, wherein X is selected from the group of CH₃, COOH, OH, NH₂, and Br and wherein a is a number from about 1 to about 3, wherein n is a number from about 12 to about 18, and wherein d is a number from about 1 to about 3. In a preferred embodiment, the functionalized ceramic body includes wherein an antibiotic is the moiety attached to the calcium aluminate containing phase.

In yet another embodiment of this invention as described herein, the functionalized ceramic body further comprises at least one of a retarder, water, an accelerator, a surfactant, a foaming agent, a fiber, a source of phosphate, an orthophosphate, and a reactive alumina, and combinations thereof.

In a more preferred embodiment of this invention, the functionalized ceramic body as described herein is functionalized with an organic acid and subsequently reacted with 3-maleimidopropionic acid N-hydroxysuccinimide ester (NHS) for binding one or more peptides to an amino-functionalized calcium aluminate containing phase.

The functionalized ceramic body may be an artificial prosthesis wherein the artificial prosthesis is selected from the group consisting of an artificial bone, artificial joint, in-vitro support structure, an in-vivo support structure, and a scaffolding matrix for support of cell, tissue, organ, and nerve growth.

In another embodiment of this invention, a functionalized composition is provided comprising a calcium aluminate containing phase that is functionalized with a linker group (linking group or coupling group) comprising an organic acid molecule for providing a reactive location for the attachment of at least one of another chemical moiety (molecule or compound)or a biologically active moiety to the calcium aluminate containing phase. The functionalized composition comprises a calcium aluminate containing phase comprising one or more phases, analogs and derivatives of calcium aluminate that result from the interaction of CnAy and CnAy-hydrates, wherein n is an integer about 12, y is an integer from about 1 to about 24, X is a phosphate moiety, C is CaO, and A is Al₂O₃.

In a preferred embodiment of this invention, the functionalized composition as described herein includes wherein the organic acid has a formula of (CO₂H)ₐCₙHH_{2n/2n-2} X_{d}, wherein X is selected from the group of CH₃, COOH, OH, NH₂, and Br and wherein a is a number from about 1 to about 3, wherein n is a number from about 12 to about 18 and wherein d is a number from about 1 to about 3.

In a more preferred embodiment of this invention, the functionalized composition as described herein includes wherein an antibiotic is the moiety attached to the calcium aluminate containing phase. The antibiotic may be any antibiotic, such as for example but not limited to ampicillin or vancomycin. The present modified compositions comprising a calcium aluminate containing phase provides for the localized delivery of antibiotic to a specific area of a patient's body. This is beneficial since amount and concentration of the localized delivered antibiotic may be increased in a manner not possible with employing the traditional systemic delivery routes of antibiotic administration such as oral medications, and intravenous and intramuscular therapies. It is well known by those skilled in the art that systemic delivery of antibiotics shall attack bacteria throughout the body including for example the bacteria flora needed for maintaining a healthy gastrointestinal tract. The modified compositions of the present invention provide for the effective delivery of antibiotics to a specific localized area of a patient's body in high concentration without adversely effecting the beneficial bacteria located in other parts of the patient's body. As used herein the term "patient" refers to all members of the animal kingdom including for example but not limited to human beings.

In yet another embodiment of this invention, the functionalized compositions as described herein comprise at least one of a retarder, water, an accelerator, a surfactant, a foaming agent, a fiber, a source of phosphate, an orthophosphate, and a reactive alumina, and combinations thereof.

In a more preferred embodiment of this invention, the functionalized composition as described herein comprises a calcium aluminate containing phase that is functionalized with the organic acid and subsequently reacted with 3-maleimidopropionic acid N-hydroxysuccinimide ester (NHS) for binding one or more peptides to an amino-functionalized calcium aluminate containing phase.

Another embodiment of this invention provides a method of making an artificial prosthesis comprising mapping of a patient's identified bone or tissue structure; creating a three dimensional pattern of the identified bone or tissue structure from the mapped bone or tissue structure; creating a mold or negative of the identified bone or tissue structure from the pattern; casting a functionalized composition (as described herein) comprising a calcium aluminate containing phase (as described herein) that is functionalized with a linker group comprising an organic acid molecule for providing a reactive location for the attachment of at least one of another chemical moiety (molecule or compound) or a biologically active moiety to the calcium aluminate containing phase, into the mold thereby forming the artificial prosthesis. In a preferred embodiment, the method as described herein includes wherein an antibiotic is the moiety attached to the calcium aluminate containing phase. The method also includes wherein said functionalized composition further comprises at least one of a retarder, water, an accelerator, a surfactant, a foaming agent, a fiber, a source of phosphate, an orthophosphate, and a reactive alumina, and combinations thereof. In a preferred embodiment of this method, as described herein, the calcium aluminate containing phase is functionalized with the organic acid and subsequently reacted with 3-maleimidopropionic acid N-hydroxysuccinimide ester for binding one or more peptides to an amino-functionalized calcium aluminate .

The following examples demonstrate the instant invention in greater detail. These examples are not intended to limit the scope of the invention in any way.

### EXAMPLE 1

| | Wt. % |
|---|---|
| Calcium Aluminate (various phases) | 99 |
| Citric Acid Monohydrate | 0.2 |
| Castament FS20 | 0.55 |
| Herculon 153 fibers | 0.25 |
| Water (for casting as a 'plus' addition) | 22.0 |

In this example an inert mold of the object would be created from the three dimensional data. Common mold materials are aluminum, steel, PVC or polyurethane. Water would be added to the above mix to give it a vibration cast consistency. This mix would then be vibrated into the mold. In other examples the water addition, additives and consistency of the material could be adjusted to allow for slip casting or gel casting. The water demand of the mixture is controlled by the particle size distribution of the mix and also the surfactant (in Example 1, Castament FS20). Examples of other surfactants are, but not limited to, sodium tripolyphosphate (STP or STPP), Darvan #7, and Melflux. As will be understood by those persons skilled in the art, the choice of surfactant shall affect the water demand and associated additive concentrations such that they will need to be adjusted, accordingly. In examples 1-4, water added was kept constant in order to compare other resulting properties. A typical water range can be from about 5% - 75%.

The material would be allowed to 'set' (precipitation of the calcium aluminate-hydrate phases). The speed of this reaction is slowed by the addition of citric acid monohydrate. Other materials that can control the reaction or 'set' time are, for example but not limited to, boric acid and anhydrous citric acid (as retarders) and lithium carbonate, sodium silicate or sodium aluminate (as accelerators).

The 'set' results in a shape exhibiting strong mechanical properties in a mechanism very similar to that of concrete. The mold would then be stripped and the shape and dried in an oven at approximately 110 degrees Celsius (C). In this form the shape would be composed of various calcium aluminate phases and calcium aluminate hydrate phases, alumina gel, alumina (present in the calcium aluminate starting material), Herculon 153 fibers (given as an example but substitution of biocompatible fiber can be accomplished). Typically this shape would now be fired at about 1000° C. During the firing process the calcium aluminate-hydrates and alumina gel will be converted to the unhydrated phases (primarily CA and CA₂) and the alumina gel will convert to the oxide. This process will also introduce porosity in place of the chemically combined water and the organic fiber. The organic fiber is introduced to allow for interconnected porosity after burn-out. The diameter of the resulting channels is determined by controlling the diameter of the starting fiber. The presence of the fiber also gives water a pathway of escape from the shape, although this is not critical in small shapes. The resulting shape is suitable as scaffoldings or as an artificial bone structure capable of supporting stem cells that will differentiate into osteoblasts (in the case of bone). In addition, this structure can now be chemically altered to accommodate binding of proteins or other bioactive factors, promoting bone growth, for example. Once introduced in vivo, the matrix will again begin to hydrate which will allow bio-decomposition to occur while natural bone is being formed. If during the firing process, the shape is exposed to temperatures from 1400 degrees C (Centigrade) to about 1650 degrees C, and preferably near 1550 degrees C, CA₆ will be formed and re-hydration will not occur. In some cases this may be desirable, for example hip replacement, where a well defined geometric structure needs to be maintained.

A variety of other compositional examples are given here with a short explanation of possible benefits.

### EXAMPLE 2

| | Wt. % |
|---|---|
| Calcium Aluminate (various phases) | 84 |
| Citric acid monohydrate | 0.2 |
| Reactive aluminas | 15 |
| STPP | 0.55 |
| Herculon 153 fibers | 0.25 |
| Water (for casting as a 'plus' addition) | 22.0 |

In this example reactive aluminas such as ALMATIS' A-2, A-3000 and A-1000 are added to give improved casting character and a denser, less porous final matrix. Herculon 153 fibers are fibrous materials commercially available from Hercules, Incorporated, Wilmington, Delaware. Darvan #7 is a sodium polymethacrylate composition used as a surfactant and is commercially available from R. T. Vanderbilt Company, Inc., Norwalk, Ct.

### EXAMPLE 3

| | Wt. % |
|---|---|
| Calcium Aluminate (various phases) | 98.5 |
| Foaming agent | 1.0 |
| Darvan #7 | 0.5 |
| Water (for casting as a 'plus' addition) | 22.0 |

In this example a foaming agent such as CF-500 or CF-700 from Unifoam, is used to introduce a high degree of porosity to the finished material. The diameter of the porosity can be controlled by the choice of foaming agent (e.g. CF-700 gives a larger bubble) and the volume of porosity is controlled by the amount of foaming agent added.

### EXAMPLE 4

| | Wt. % |
|---|---|
| Calcium Aluminate (various phases) | 90.5 |
| Foaming agent | 1.0 |
| Calcium orthophosphate | 8.0 |
| Melflux | 0.5 |
| Water (for casting as a 'plus' addition) | 22.0 |

In this example a phosphate source is added to give raw material for osteoblast precipitation of natural bone. Melflux is a polymeric surfactant commercially available from Degussa Construction Polymers, Kennesaw, GA.

As can be seen in these examples there are a variety of strategies one can take in determining an appropriate starting matrix. The examples set forth herein are given to demonstrate this breadth, however, they are not intended to limit the scope of the present invention as described herein. These examples set forth herein are for the purposes of illustration and it will be evident to those persons skilled in the art that numerous variations and details of the instant invention may be made without departing from the instant invention as set forth herein.

### DETAILED COMPOSITIONAL STRATEGY, EXAMPLE 5

Example 5 will be used to demonstrate a detailed compositional matrix and the resulting physical properties of the resulting solid body.

Calcium Aluminate Clinker of the following chemistry (reported on an oxide basis) was obtained for the study. The material was screened, sized and chemistry was determined on each fraction (see table I)

| Oxide | Fraction | | | |
|---|---|---|---|---|
| | +10m | 10/28m | 28/65m | -65m |
| | (concentration in Wt%) | | | |
| SiO2 | 0.44 | 0.29 | 0.22 | 0.25 |
| Al2O3 | 71.59 | 71.21 | 70.35 | 71.19 |
| Fe2O3 | 0.07 | 0.01 | <0.01 | 0.01 |
| CaO | 27.38 | 28.08 | 29.02 | 27.95 |
| MgO | 0.27 | 0.22 | 0.21 | 0.31 |
| Na2O | 0.23 | 0.17 | 0.18 | 0.26 |
| K2O | 0.01 | 0.01 | 0.01 | 0.02 |
| P2O5 | 0.01 | 0.01 | 0.01 | 0.01 |

Mineralogical Examination of these fractions showed the following:

| Compound | +10m | 10/28m | 28/65m | -65 |
|---|---|---|---|---|
| | Present | | | |
| CaAl2O4 (CA) | M | M | M | M |
| CaAl4O7 (CA2) | M | M | M | M |
| Cal2Al14O33 (C12A7) | m | m | t | nd |
| Ca3Al2O6 (C3A) | nd | nd | nd | nd |
| Ca5Al6O14 (C5A3) | nd | nd | nd | nd |
| Ca2Al12O5 (C2A) | nd | nd | nd | nd |
| CaAl12O19 (CA6) | nd | nd | nd | nd |
| Ca3Al10O18 (C3A5) | nd | nd | nd | nd |
| CaO (C) | t | t | t | t |
| Al2O3 (A) | t | t | t | t |

| | | | | |
|---|---|---|---|---|
| M = Major, m = minor, t = trace, nd = not detected | | | | |

This chemistry and mineralogy is typical for a 70% alumina containing calcium aluminate cement, calcium aluminate cements containing greater than 70% alumina can be used. Calcium aluminate cement containing less than 70% alumina can also be used; however, most commercially available products have impurities, which increase in concentration as the alumina content decreases. Common brands of 70% alumina containing calcium aluminate cement are ALAMITIS' "calcium aluminate 14" product and Lafarge's Secar 71 product.

The typical average open porosity of the CₙA_{y} aggregate is 53.5% while the TSG (typical specific gravity) averages 2.9 g/cm³.

**COMPOSITION EXAMPLE 5****

| Aggregate | Wt% |
|---|---|
| CₙA_{y} +10m | 15% |
| CₙA_{y} 10/28m | 30% |
| CₙA_{y} 28/65m | 10% |
| CₙA_{y} -65m | 11% |
| CₙA_{y} -325m | 7% |
| A-2 alumina | 8% |
| A-3000 alumina | 10% |
| A-1000 alumina | 9% |
| STPP (plus addition) | 0.15% |

| | |
|---|---|
| ** Example 5 is a calcium aluminate (CₙA_{y}) composition of the instant invention comprising a blend of CA (50% wgt.) and CA₂ (50% wgt.). | |

24% by weight of water was added to give a vibration cast consistency. The material was cast into simple bars in order to determine modulus and crushing strengths. The shape was stripped from the mold in 24 hours and dried at 110°C. Finally, the shape was fired to a temperature of 1100°C and allowed to reach thermal equilibrium. The shape was allowed to cool and was tested. The results are as follows:
Apparent porosity = 50%
Average pore size = 44 microns
Cold crushing strength (ASTM C133) = 34.5 MPa
Modulus of Rupture (ASTM C133) = 9.3 MPa

### EXAMPLE 6

### MATERIALS AND METHODS

### 6.1 Human MG-63 cell culture

MG-63 cells (ATCC,CRL-1427) at passage 88 were cultured in F12 Ham/Minimal Essential Medium (1:1, Gibco) with 10% fetal bovine serum (Gibco, 10082-139) and penicillin streptomycin (Gibco) at 37°C with 5% CO₂ and 90% humidity. Cells were passaged when sub-confluent using trypsin/EDTA (Gibco,25300-054), and between passages 88-98.

### 6.2 Human Adult Mesenchymal Stem Cell (hAMSC) culture

Bone-marrow derived human adult mesenchymal stem cells (Cambrex,PT-2501) were cultured in mesenchymal stem cell growth media (MSCGM, Cambrex,PT-3001,PT-3238) at 37°C with 5% CO₂ and 90% humidity. When sub-confluent, cells were released using trypsin/EDTA and used between passages 3-10. For differentiation, hAMSC were cultured in medium with an osteogenic supplement (Cambrex,PT-3002) containing beta-glycerophosphate, ascorbic acid, and dexamethosone.

### 6.3 Preparation of calcium aluminate materials

Compositions comprising a calcium aluminate containing phase were obtained from Westmoreland Advanced Materials Bioceramics, Inc. (Monessen, PA, USA). Calcium aluminate of approximately 0.85-2mm [herein the size of about 0.85mm to about 2 mm (millimeter) is referred to as a "morsel"] were collected by sieving and sterilized by autoclaving.

### 6.4 Seeding of cells on calcium aluminates for static culture

One gram of sterile calcium aluminate morsels were placed into each well of a 12-well plate and 1 x 10⁵ MG-63 cells or 1 x 10⁴ hAMSC were added per well in a total volume of 3-4mls medium. After 24 hours in culture, the calcium aluminate morsels were washed to remove any unattached cells, transferred to a new well plate, and the medium was replaced.

### 6.5 Seeding of cells on calcium aluminates for dynamic culture

Three-gram samples of sterile calcium aluminates were placed into the bottom of a spinner flask (Bellco Glass, 7761-00100). A total of 2.5 x 10⁶MG-63 cells (ATCC, CRL-1427) at passage 88, or 1.2-1.3 x 10⁶hAMSC were added in a volume of 60mls of appropriate medium (F12 Ham/Minimal Essential Medium [1:1, Gibco] with 10% fetal bovine serum [Gibco, 10082-139] and penicillin streptomycin [Gibco] at 37 degrees Centigrade with 5%CO₂ and 90% humidity) with stirring at 17-20 rpm. After 24 hours in this culture, the calcium aluminate morsels were washed to remove any unattached cells, transferred to new spinner bottles, and the medium was replaced.

### 6.6 Cell Quantitation by CyQuant Cell Proliferation Assay Kit

Attachment and proliferation of MG-63 cells or hAMSC on calcium aluminates were assessed by the CyQuant assay (Molecular Probes, C-7026). Calcium aluminates samples with cells attached were washed three times with PBS to remove unattached cells, and then stored at -80° C. Samples were subjected to several freeze/thaw cycles, and then assayed as designated by the manufacturer. Each sample is assayed by measuring the fluorescence (480nm excitation and 500nm emission) in a Perkin Elmer HTS 7000 Bio Assay Reader. Standard curves were used to convert fluorescence units into cell numbers.

### 6.7 Viability and Cell Quantitation by Live/Dead Fluorescence Staining

Viability and attachment of cells on the calcium aluminate morsels were assessed with the LIVE/DEAD Viability/Cytotoxicity Assay kit (Molecular Probes, L-3224). Calcium aluminate samples with cells attached were washed three times with PBS and then stained with the Live/Dead fluorescent dye as suggested by the manufacturer. The Live/Dead stained samples were viewed using a fluorescent microscope (Nikon Eclipse E600), and viability was determined. Ten morsels of calcium aluminate were chosen at random and the live and dead cells were counted.

### 6.8 Cell Attachment and Morphology by Scanning Electron Microscopy

Calcium aluminate samples of the present invention were washed three times with phosphate-buffered saline (PBS) and fixed in 2.5% gluteraldehyde in PBS. The samples were washed with PBS, stained with osmium tetroxide, washed with water, dehydrated through an ethanol series, and critical point dried. Samples were mounted, gold-coated, and viewed with a Hitachi 2460 scanning electron microscope at an accelerating voltage of 5kV or 15kV. Digital images were captured at varying magnifications.

### 6.9 Alkaline Phosphatase Histochemical Staining

Reagents were prepared using the alkaline phosphatase histochemical staining kit (Sigma 85-4C). Samples were washed three times with PBS, fixed for one minute with citrate-buffered methanol, washed three times with distilled water, and stained as described by the manufacturer.

### 6.10 Alkaline Phosphatase Biochemical

Samples were washed three times with PBS and stored at -80°C. To prepare lysates, samples were subjected to a series of freeze/thaw cycles and vortexed. Lysates were clarified by centrifuge in a microfuge (12,000 x g for 10 seconds) and the supernatant was assayed in triplicate for alkaline phosphatase activity. The buffer solution was prepared by mixing equal volumes of 2-amino-2-methyl-1-propanol (Sigma A9879), p-nitrophenol phosphate (Sigma 104-0), and 1M MgCl₂. A standard curve of p-nitrophenol was used to estimate p-nitrophenol produced. Alkaline phosphatase activity was calculated as pmol p-nitrophenol produced per microgram of protein in cell lysate per hour. Absorbances were read at 405nm on a BioRad Model 3550 Microplate Reader. Protein concentration was estimated using the BCA Protein Assay (Pierce Biotechnology,23225) using bovine serum albumin to generate standard curves.

### 6.9 Chick chorioallantoic membrane (CAM) assay

Fertilized White Leghorn chicken eggs were incubated for three days at 100 °F and 70% relative humidity. The eggs were cracked into 100 mm X 20 mm sterile cell culture dishes containing 4 mL of sterile Dulbecco's Modified Eagle Media (InVitrogen) with 1.5 g/L sodium bicarbonate. Embryos were incubated at 38 °C for an additional 7 days to allow for the CAM to fully develop. Morsels of calcium aluminate were applied to CAMs at day 10 and incubated for 72 hrs. Immediately prior to imaging, the vasculature was labeled with Qdots by injection of 10-20 µl 2-8 mM Qdots into the large vitelline vein using glass needles with ∼100 µm diameter bore. Light and fluorescence imaging to measure angiogenic response was performed immediately post injection using an inverted Zeiss Axiovert 135TV microscope equipped with a Hamamatsu ORCA II cooled CCD camera. Fluorescence images of quantum dots were acquired with a 450/50 nm excitation filter and appropriately selected 20nm wide bandpass emission filters, centered on the emission maxima of the quantum dots.

### 6.10 Statistical Analysis

The Vassar Stats online statistical program (http://faculty.vassar.edu/lowry/VassarStats.html) was used to calculate means, standard errors, analysis of variance (ANOVA), Tukey's *post hoc* tests, and t-tests. ANOVA and the Tukey *post hoc* test were used to compare multiple means. Statistical significance was p<0.05.

### RESULTS

### MG-63 cells attach on calcium aluminates proliferate, and maintain high viability under dynamic and static seeding and culture conditions.

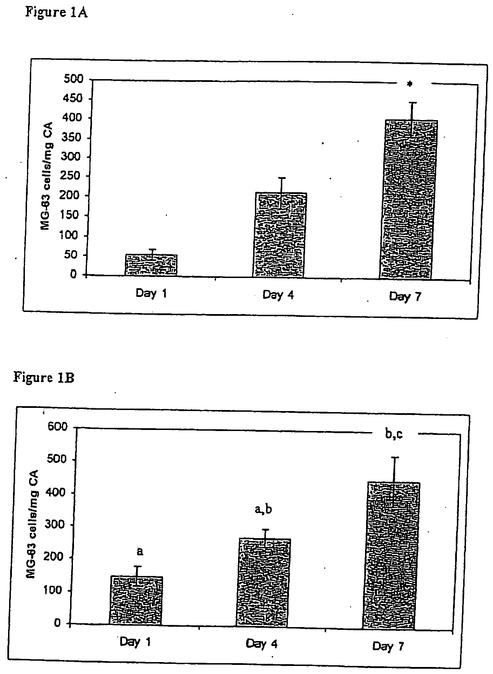

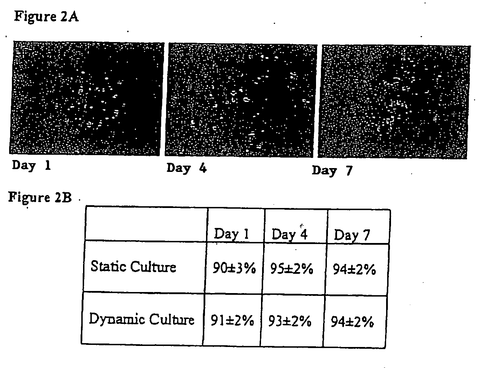

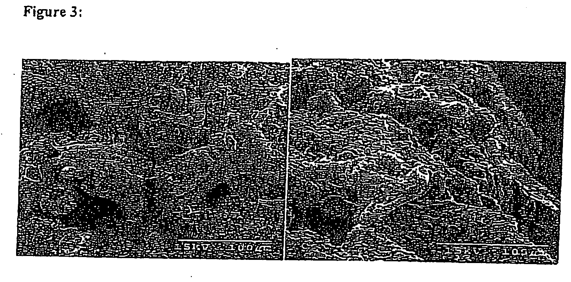

To assess the attachment and proliferation of MG-63 cells on calcium aluminates of the present invention under dynamic culture conditions, a fixed amount of calcium aluminate (3.0 g per flask) was incubated in spinner flasks with 2.5 x 10⁶ MG-63 cells. For static conditions, a fixed amount of calcium aluminate (1.0 g per well) was incubated in a 12-well plate with 1.1 x 10⁵ MG-63 cells per well. The CyQuant assay was used to determine attachment and proliferation of MG-63 cells on the calcium aluminate at days 1, 4, and 7. Samples taken on day 1 represent initial MG-63 cell attachment on the calcium aluminate. Samples were then washed to remove unattached cells after day 1, and assayed on days 4 and 7 of culture to assess proliferation. Under dynamic culture conditions, cell numbers increased over the one-week period as assessed by CyQuant assay with a statistically significant increase (p < 0.05) between days 1 and 7 determined by ANOVA and Tukey's *post hoc* test (Figure 1A). This statistically significant increase was observed in four independent experiments. Under static culture conditions, there was a statistically significant increase (p < 0.05) consistently observed in three independent experiments between days 1 and 7, and days 4 and 7 as determined by ANOVA and Tukey's *post hoc* test (Figure 1B). Throughout the one week period examined, the viability of MG-63 cells under dynamic or static culture conditions is 90% or higher, as assessed by Live/Dead fluorescent staining (Fig 2). Scanning electron microscopy showed that MG-63 cells produce an extracellular matrix, attach, and spread over the surface of the calcium aluminate (Fig. 3).

### Human adult mesenchymal stem cells (hAMSC) attach on calcium aluminates and show variability in proliferation under static and dynamic culture conditions.

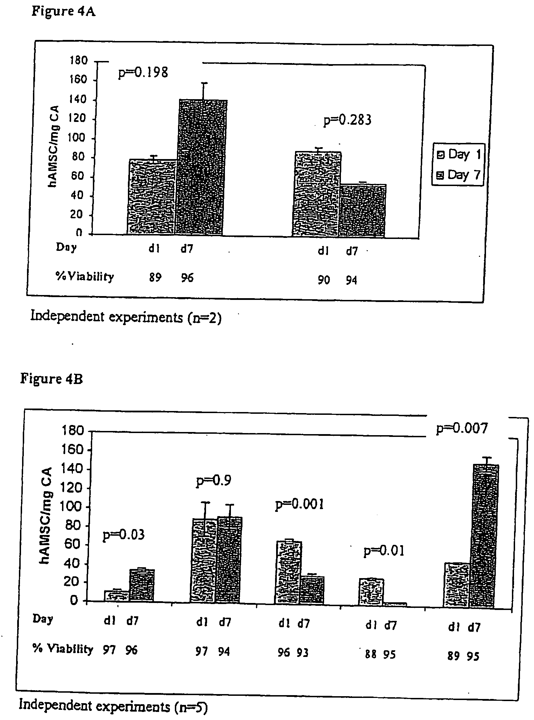

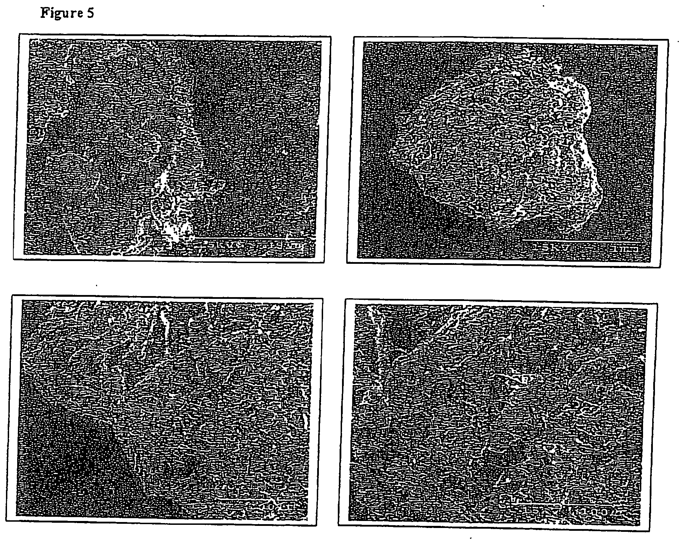

To assess the attachment and proliferation of hAMSC on calcium aluminate, a fixed amount of calcium aluminate (1.0 g per well for static cultures; 3.0 g / spinner bottle for dynamic cultures) was seeded with human adult mesenchymal stem cells (1 x 10⁴ cells per well for static cultures; 1.2 x 10⁶ cells per spinner flask for dynamic cultures). Samples taken and assayed by the CyQuant assay on day 1 represent initial hAMSC attachment on the calcium aluminate. Samples were then washed to remove unattached cells after day 1, and assayed by CyQuant on days 4 and 7 of culture to assess proliferation. Human adult mesenchymal stem cells are able to attach to calcium aluminate after 24 hours in static or dynamic culture as assessed by the CyQuant assay. Figure 4 presents the results of two independent and representative experiments under static culture conditions (Figure 4A) and five independent experiments under dynamic conditions (Figure 4B). There was variability in the proliferation of hAMSC among the experiments under static and dynamic culture conditions. In some cases there was a statistically significant increase in cell numbers over the seven day culture period, in some cases there was a statistically significant decrease, and in some cases there was no difference. In all of the experiments, those cells attached to the calcium aluminate maintained viability over 90% under either static or dynamic culture (Figure 4A and B). Scanning electron microscopy of hAMSC cultured on the calcium aluminate through three weeks of culture show cell attachment, though the cells do not appear to be as spread over the surface as is typical for these cells on tissue culture plastic (Figure 5).

### Differentiation of human adult mesenchymal stem cells on calcium aluminates

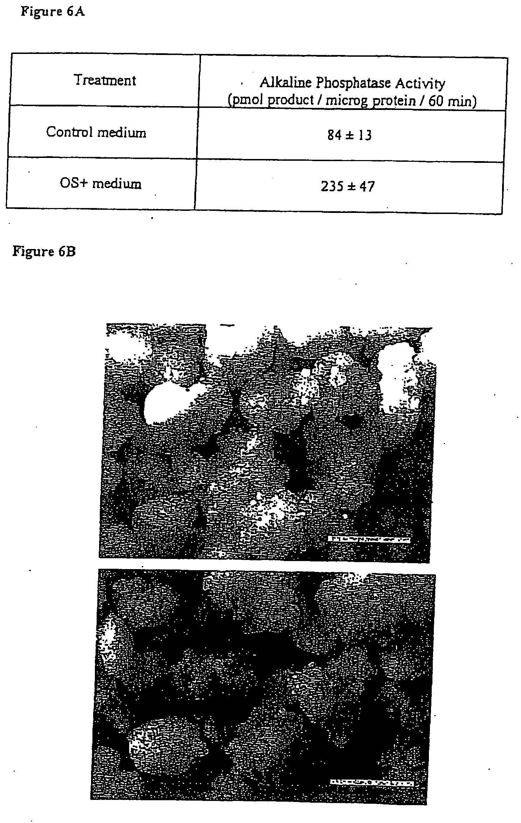

To assess the differentiation of human adult mesenchymal stem cells into osteoblasts, hAMSC attached to the calcium aluminate were cultured for 14 days in medium with an osteogenic supplement (Cambrex, PT-3002) containing beta-glycerophosphate, ascorbic acid, and dexamethasone or in control medium. Bone-marrow derived human adult mesenchymal stem cells (Cambrex, PT-2501) were cultured in mesenchymal stem cell growth media (MSCGM, Cambrex, PT-30001, PT-3238) at 37 degrees Centigrade (C) with 5% CO2 and 90% humidity. When sub-confluent, the cells were released using trypsin/EDTA and used between pasages 3 to 10. The levels of induced alkaline phosphatase under both conditions were assessed biochemically and histochemically (Figure 6A and B, respectively). Based on three independent experiments, hAMSC on the calcium aluminate cultured with osteogenic supplement-containing medium show a statistically significant increase (p = 0.04) in alkaline phosphatase biochemical activity relative to controls, consistent with osteoblast differentiation. Histochemical staining for alkaline phosphatase (Figure 6B) confirms the results from the biochemical assay: alkaline phosphatase staining is readily detected on numerous morsels in the OS-treated samples, and little staining is detected in the controls, even though cells were present on the control samples as detected by Live/Dead staining (data not shown).

### Calcium aluminate is non-toxic in vivo as assessed by chick chorioallantoic membrane (CAM) assays

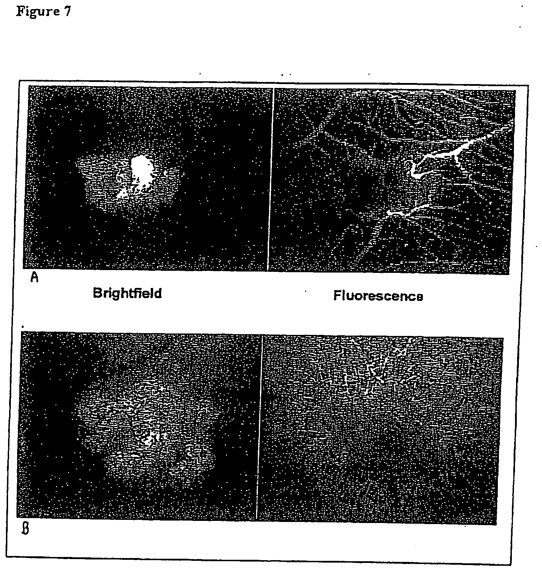

To assess calcium aluminate toxicity using an *in vivo* assay, we employed the chick embryonic chorioallantoic membrane assay (REFS). Morsels of the calcium aluminate, or clinically relevant calcium phosphate materials (ChronOS) as controls, were placed on chick CAMs and the formation of blood vessels in the vicinity of the calcium aluminate implant was assessed by fluorescent microscopy. Figure 7 shows that calcium aluminate has no obvious negative effect on the vascularization of the chick CAM. Toxic and negative effects could be evidenced in the embryo's death, the material causing severe inflammation, or neighboring blood vessels regression/death. None of these appeared to have occurred.

### FIGURE LEGENDS

**Figure 1****. MG-63 cells attach and proliferate on calcium aluminates under static (A) and dynamic (B) culture conditions as assessed by CyQuant assay.** For dynamic culture, a fixed amount of calcium aluminate (3.0 g per flask) was incubated in spinner flasks with 2.5 x 10⁶MG-63 cells. For static culture, a fixed amount of calcium aluminate (1.0 g per well) was incubated in a 12-well plate with 1.1 x 10⁵ MG-63 cells per well. The CyQuant assay was used to determine attachment and proliferation of MG-63 cells on calcium aluminate at days 1, 4, and 7. Samples taken on day 1 represent initial MG-63 cell attachment on calcium aluminate. Samples were washed to remove unattached cells after day 1, and assayed on days 4 and 7 of culture to assess proliferation. For static culture (A), each bar represents the mean and standard error of the mean of three wells from a representative experiment. Three independent experiments were performed. There is a statistically significant increase in MG-63 cell numbers between days 1 and day 7 (p<0.01), and days 4 and 7 (p<0.05) determined by ANOVA and Tukey post-hoc tests, consistent with proliferation. This statistically significant difference was in common among the three independent experiments. For dynamic culture (B), each bar represents the mean and standard error of the mean of three samples taken from the spinner flask at each time point from a representative experiment. Four independent experiments were performed. There is a statistically significant increase in MG-63 cell numbers between days 1 and day 7 (p<0.05) determined by ANOVA and Tukey post-hoc tests, consistent with proliferation. This statistically significant difference was in common among all four independent experiments.

**Figure 2****. MG-63 cells maintain high viability when seeded and cultured on calcium aluminates.** MG-63 cells seeded and cultured on calcium aluminates over a period of seven days under static and dynamic conditions were assessed for viability by Live/Dead staining. Fluorescent micrographs (A) are representative of a dynamic culture on days 1, 4, and 7. Data in B are the means and standard errors of the mean of percent viabilities of four independent experiments. For each independent experiment, live and dead cells on ten pieces of calcium aluminate were counted under fluorescent microscopy from each of three wells (static) or three samples (dynamic).

**Figure 3****. MG-63 cells attach on calcium aluminates as assessed by scanning electron microscopy.** MG-63 cells cultured under static conditions for four days were observed by scanning electron microscopy.

**Figure 4****. Human adult mesenchymal stem cells attach and show high viability but variable proliferation on calcium aluminates under static (A) and dynamic (B) culture conditions**. For static culture, a fixed amount of calcium aluminate (1.0 g per well) was seeded with human adult mesenchymal stem cells (1 x 10⁴ cells per well) in 12-well plates. For dynamic culture, a fixed amount of calcium aluminate (3.0 g / spinner bottle) was seeded with 1.2 x 10⁶ hAMSC per spinner flask. Samples were taken and assayed by the CyQuant assay on day 1 represent to assess initial hAMSC attachment on calcium aluminate. Calcium aluminate morsels were then washed to remove unattached cells after day 1, and assayed by CyQuant on day 1 and day 7 of culture to assess attachment and proliferation. For static cultures (A), the results of two independent and representative experiments (1-4) are shown. Each bar represents the mean and standard error of the mean of three wells. For dynamic cultures (B), the results of five independent experiments (1-6) are shown. Each bar represents the mean and standard error of the mean of three replicate samples from each a spinner flask. A two-tailed t test was used to assess statistically significant differences between means, and the p-values are shown above each independent experiment on the graph. The viability of attached cells as assessed by Live/Dead staining on each day for each experiment is also shown. For each independent experiment, live and dead cells on ten pieces of calcium aluminate were counted under fluorescent microscopy from each of three wells per 12-well plate (static, A) or three samples per spinner flask (dynamic, B).

**Figure 5****. Human adult mesenchymal stem cells attach on calcium aluminates as assessed by scanning electron microscopy.** hAMSC cultured under dynamic conditions for 7 days (left) and 21 days (right) were observed by scanning electron microscopy.

**Figure 6****. hAMSC on calcium aluminate express alkaline phosphatase in response to an osteogenic** supplement. hAMSC attached to calcium aluminate were cultured for 14 days in medium with an osteogenic supplement (OS+, containing beta-glycerophosphate, ascorbic acid, and dexamethasone) or in control medium. The levels of induced alkaline phosphatase under both conditions were assessed biochemically (A) and histochemically (B). The biochemical data are the mean and standard error of the mean of three independent experiments. There is a statistically significant increase (p=0.04) in alkaline phosphatase biochemical activity in OS-treated cells relative to controls, thereby consistent with osteoblast differentiation. Histochemical staining for alkaline phosphatase (B) shows that alkaline phosphatase staining is readily detected on numerous morsels in the OS-treated samples, and little staining is detected in the controls, even though cells were present on the control samples as detected by Live/Dead staining (data not shown). Scale bar = 1.25 mm.

**Figure 7****. Calcium aluminate is non-toxic as assessed by chick CAM** assays. Morsels of calcium aluminate (A) and ChronOs (control, B) were placed on the chorioallantoic membrane of embryonic chicks. One day later the embryos were injected with 8 microM 705 nm emission Qdots that were conjugated with 2000 MW PEG. Brightfield (left) and fluorescent (right) images were captured and show that the developing vasculature around the CA shows no indication of inhibition/toxicity.

As will be appreciated by those persons skilled in the art, this study (Examples 6-9) assessed the calcium aluminates of the present invention to determine their ability to support cell growth in static and dynamic culture conditions. After calcium aluminates were assessed through *in-vitro* studies, we conducted *in-vivo* studies through the CAM assay to determine the biocompatibility of calcium aluminate. Calcium aluminates were chosen due to their advantages when compared to other synthetic materials. Most forms of calcium aluminate will hydrate and form a bonding matrix. As a result, a three dimensional structure can easily be created that will exhibit high strengths (high degree of tensile stress and compressive stress resistance) without the need for thermal treatment, such as with calcium phosphates, for example but not limited to calcium orthophosphate, which are more brittle, difficult, and expensive to manufacture. In addition to providing a physically superior bond mechanism, hydrated calcium aluminate is easily metabolized by the body and overtime is replaced with natural bone. If desired, calcium aluminate can be treated so that it will not hydrate and thus will remain as a permanent support structure. Another advantage of calcium aluminate is that by carefully choosing the ratio of reactants to form calcium aluminate, the type of calcium aluminate formed can be controlled along with the resulting porosity, surface structure, and solubility of the material. As mentioned briefly, this may allow a degree of cell attachment control as this potentially relates to surface structure. By choosing the ratio of reactants to form calcium aluminate the type of calcium aluminate that forms can be controlled, along with the resulting porosity and solubility of the material.

Some synthetic materials, such as the forms of calcium aluminates, which we have investigated, have a natural porosity due to their synthetic mechanism. This permits the necessary vascularization and hence bond between the implant and natural bone. In addition, the hardness of the CA material and natural bone are very similar and therefore abrasion loss is drastically reduced. Therefore, synthetic ceramics such as calcium aluminate should be able to replace metal implants and be more advantageous to long term and stable medical implants and the industry supporting this technology.

Calcium aluminates are composed of hydratable compounds that add strength to the material via a dissolution/precipitation reaction when mixed with water. Porosity is able to be controlled and the material can be molded into any desirable shape. Calcium aluminate can be engineered to match the desired finished structure, and, in addition, in the form of hydrates, these materials will slowly be metabolized by the body. There is limited research available on calcium aluminates for use in tissue engineering. From the limited research, there have been few *in-vivo* studies. However, Klawitter and Hulbert were able to determine that calcium aluminates do not cause an inflammatory response when implanted into dog femurs.

In this study (examples 6-9) we have shown that calcium aluminate material of the present invention are able to support the growth and high viability of two types of cells; the human adult mesenchymal stem cells and MG-63 osteosarcoma cells. MG-63 cells attach and proliferate in dynamic and static culture conditions over the course of 7 days. The MG-63 cells also maintain high viability at or above 90% during the 7 days in both types of culture. Human adult mesenchymal stem cells (hAMSC) attach in dynamic and static culture conditions. The hAMSC maintain high viability at or above 90% throughout 7 days in both cultures. Proliferation is variable with the hAMSC in both culture conditions. Another possible reason for the observed variation may have been due to the inconsistency of hAMSC, which were purchased at different times thereby resulting in different demographic characteristics based on the source of the human adult mesenchymal stem cells. However, even with the variability with the hAMSC proliferating on calcium aluminate, the cells are able to differentiate into osteoblasts and express alkaline phosphatase activity.

Furthermore, we have assessed the biocompatibility of calcium aluminates through *in-vivo* chick chorioallantoic membrane (CAM) assays. We have determined that the calcium aluminate compositions of the present invention do not cause a toxic response to the chick membranes, which is consistent with our *in-vitro* results. Based on the *in-vitro* studies involving the attachment and proliferation of human adult mesenchymal stem cells and MG-63 cells, *in-vitro* differentiation studies ofhAMSC, and the *in-vivo* CAM assays, we believe calcium aluminate materials may be an effective material for bone regenerative medicine.

### EXAMPLE 7

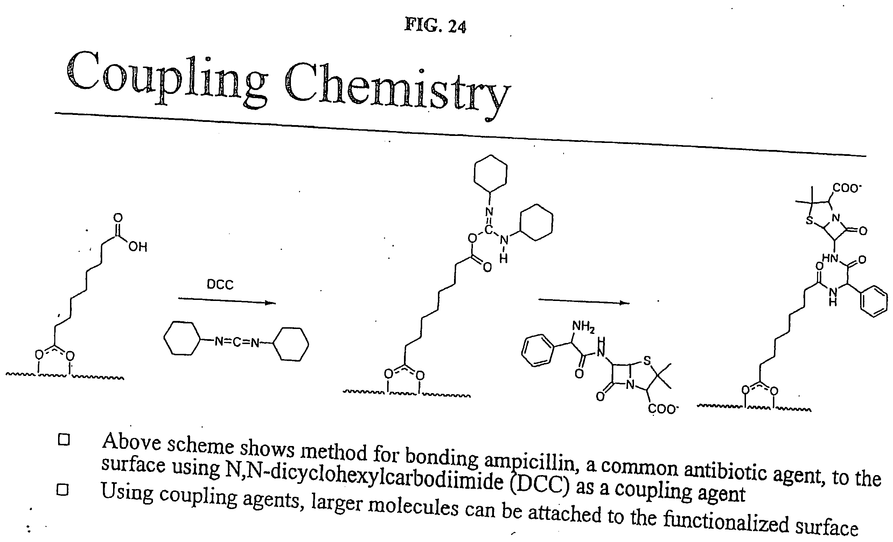

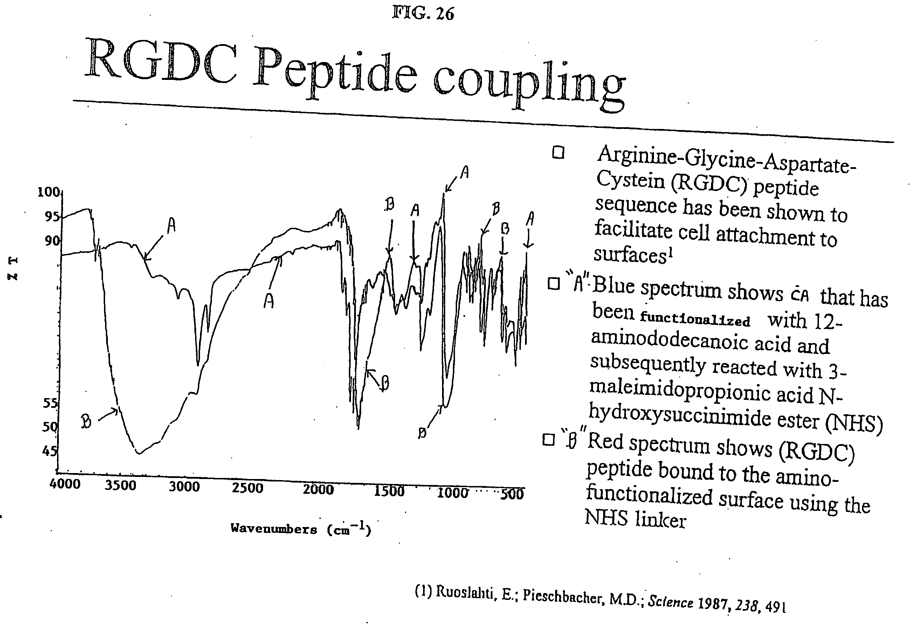

The following procedures were followed to functionalize the ceramic body and/or the calcium aluminate compositions of the present invention. As used herein the terms "functionalize" and/or "functionalization" refer(s) to the modification of the ceramic body and/or the compositions comprising a calcium aluminate containing phase as described herein of this invention by the deposition or attachment of an organic acid to the ceramic body and/or the calcium aluminate containing phase of the compositions of this invention. The functionalization forms a functionalized interface between the ceramic body or the calcium aluminate containing phase of the compositions of this invention and the biological tissue which results in an improvement of the ceramic body's or the calcium aluminate containing composition's biocomptability and/or resistance to infection. Figures 19-26 show examples of the functionalization of the ceramic body and/or calcium aluminate containing compositions of the present invention with organic acids. Figure 24 shows the coupling chemistry for the attachment of an antibiotic to the ceramic body and/or calcium aluminate containing compostions of this invention. Figure 26 shows the coupling of a peptide sequence to an amino-functionalized calcium aluminate containing composition of this invention wherein calcium aluminate was functionalized with 12-aminododecanoic acid and subsequently reacted with 3-maleimidopropionic acid N-hydroxysuccinimide ester (NHS) as linking agents (coupling agents). The morsels of calcium aluminate containing compositions set forth in the following functionalization (deposition) examples were obtained from Westmoreland Advanced Materials Bioceramics, Inc. (Monessen, PA, USA). The composition of the calcium aluminate that was employed for the deposition and attachment examples set forth below was primarily a CA (50% by weight) and CA₂ (50% by weight) composition of the present invention. It will be understood by those skilled in the art that any of the compositions comprising a calcium aluminate containing phase of this invention as described herein may be employed as the composition for attaching the linker and any desired chemical and biologic moiety. The CA and CA₂ composition was chosen merely as an example and is not to be construed to limit the scope of the present invention. It is important to note that when the linker group is an acid the head group and the tail group of the acid molecule may be varied to have varying functionalities in order that the ceramic body or the calcium aluminate containing compositions being modified provide one or more reactive locations for the attachment of other chemical molecules or biological moieties.

### 7.1 Octadecylphosphonic acid deposition

ImM solution of octadecylphosphonic acid was prepared in dry THF (tetrahydrofuran). Morsels of calcium aluminate compositions of this invention were placed into this solution at room temperature. After remaining in the solution for one hour the calcium aluminate compositions were removed from the solution and placed in a glass dish and dried in the oven at 120°C overnight, approximately 12 hours.

### 7.2 Stearic Acid (octadecanoic acid) deposition

ImM solution of stearic acid was prepared in dry distilled THF. Morsels of calcium aluminate containing compositions of this invention were placed in this solution for 1 hour at room temperature. The calcium aluminate containing compostions were then removed from the solution and placed in a 120°C for 12 hours to dry.

### 7.3 12-bromododecanoic acid deposition

Morsels of CA containing compositions of this inventions were placed in a 2mM solution of 12-bromododecanoic acid in THF for 1 hour at room temperature. The calcium aluminate containing compositions were removed from solution and dried in the oven at 120°C for 12 hours.

### 7.4 12-aminododecanoic acid deposition

A small amount of 12-aminododecanoic powder was placed in dry THF and sonicated for 5 minutes to form a saturated solution. Morsels of calcium aluminate containing compositions of this invention were dipped in the saturated 12-aminododecanoic acid solution for 1 hour at room temperature. The calcium aluminate containing compositions were removed from solution and placed in oven at 120°C for 12 hours.

### 7.5 1,12-dodecanedicarboxylic acid deposition

Morsels of calcium aluminate containing compositions of this invention were dipped in a ImM solution of 1,12-dodecanedicarboxylic acid for 1 hour at room temperature. The calcium aluminate containing compositions were then removed from solution and dried in the oven for 12 hours at 120°C.

### 7.6 DCC/NHS linking

Morsels of calcium aluminate containing compositions of this invention that have been functionalized with 1,12-dicarboxylic acid were placed in a solution of 2mM N-hydroxysuccinimide and 2mM N,N-dicyclohexylcarbodiimide(DCC) for 24 hours. The calcium aluminate containing compositions were removed from solution and placed under vacuum on a schlenk line (0.1 torr) for 1.5 hours to dry.

### 7.7 Ampicillin or vancomycin attachment.

Morsels of calcium aluminate containing compositions of this invention that had been functionalized with the NHS linker were dipped in 1mg/ml solutions of either ampicillin or vancomycin in methanol immediately after removal from vacuum tube. The calcium aluminate containing compositions were left in solution for 24 hours at 30°C . The calcium aluminate containing compositions were then removed from solution and dried under vacuum in a vacuum tube on a schlenk line (0.1 torr).

### 7.8 RGDC Peptide linking

Morsels of calcium aluminate containing compositions of this invention that had been functionalized with 12-aminododecanoic acid were dipped in a solution of ImM 3-maleimidopropionic acid N-hydroxysuccinimide ester (NHS) in acetonitrile for 24 hours at room temperature. The calcium aluminate containing compositions were then removed from solution and placed under vacuum for 24 hours to dry. The calcium aluminate containing compositions were then dipped in 1mg/ml aqueous solution of RGDC peptide (arginine-gycine-aspartate-cystein) for 24 hours. The calcium aluminate containing compositions were again removed from solution and placed under vacuum (0.1 torr) for 24 hours.

### EXAMPLE 8

In this example, modified calcium aluminate morsels described in example 7.2 were taken to form a cast shape. Modified morsels of the appropriate size distribution (set forth below) were blended with -325 mesh calcium aluminate to form a composition. The size distribution of this composition was 50% (wt) -325 mesh, 25% (wt) -10+60 mesh and 25% (wt) -60 mesh. To this composition 20% (wt) water was added to form a vibratable castable which was formed into a 1 x 1 x 5" bar using a Teflon coated mold. The composition was allowed to set and then cured for 24 hours. It was then analyzed to determine that the stearic acid linker modification was still intact, which it was. This example is a basis for a prosthetic device formed of the functionalized calcium aluminate where the modification is throughout the prosthetic and is available as the prosthetic is metabolized. This strategy allows the applicants of the present invention to modify the calcium aluminate morsels with different functional groups thereby giving the present applicants the ability to target two or more biological and/or chemical processes.

### EXAMPLE 9

In this example calcium aluminate morsels were blended to provide the appropriate size distribution (set forth below) for a vibration castable. The size distribution of this composition was 50% (wt) -325 mesh, 25% (wt) -10+60 mesh and 25% (wt) -60 mesh. To this composition 25% (wt) water was added to form a vibratable castable which was formed into a 1 x 1 x 5" bar using a Teflon coated mold. The composition was allowed to set and then cured for 24 hours. This entire shape was then treated according to example 7.2 in order to functionalize the surface and near surface (due to porosity) of the bar. The bar was analyzed to determine successful stearic acid functionalization. This was the case. This example is the basis for a prosthetic device formed of the functionalized calcium aluminate of the present invention where the modification would be only on the surface or near surface of the prosthetic. Addition of an antibiotic functional group, as described herein, allows for the potential attack of the formation of biofilms on the surface of the prosthetic device. The present modification may be used as a barrier to tissue attachment to treat the inside of a skull plate or implant to successfully prevent tissue attachment to the inside of the skull.

It will be appreciated by those persons skilled in the art that the linker groups disclosed herein employed to functionalize the ceramic bodies and/or compositions comprising a calcium aluminate containing phase of this invention bind well to the calcium aluminate containing compositions. Those persons skilled in the art shall understand that the present applicants have shown that the calcium aluminate containing compositions of this invention may be functionalized with various head and tail groups on long chain carboxylic acids in order to provide for further chemical or biological modification of the calcium aluminate containing compositions. The present applicants have successfully functionalized a ceramic body and a composition containing a calcium aluminate containing phase, as described herein, with antibiotics and peptide molecules.

Whereas particular embodiments of this invention have been described above for purposes of illustration, it will be evident to those persons skilled in the art that numerous variations of the details of the present invention may be made without departing from the invention as defined herein and in the appended claims.

## Claims

1. A functionalized ceramic body comprising:
a calcium aluminate containing phase that is functionalized with a linker group comprising an organic acid molecule for providing a reactive location for the attachment of another chemical moiety or a biologically active moiety to the ceramic body,
wherein said organic acid molecule has a formula of (CO₂H)ₐCₙH_{2n/2n-2} X_{d},
wherein X is selected from the group of CH₃, COOH, N, N-dicyclohexylcarbodiimide (DCC)-coupled COOH, OH, NH₂, a phosphate moiety and Br, wherein a is a number from 1 to 3, wherein n is a number from 12 to 18, and wherein d is a number from 1 to 3,
said reactive location being subsequently reacted with said other chemical moiety or said biologically active moiety to attach said other chemical moiety or said biologically active moiety to said functionalized calcium aluminate containing phase.

2. The functionalized ceramic body of Claim 1 wherein the calcium aluminate containing phase comprises one or more phases of calcium aluminate that result from the interaction of CₙA_{y} and CₙA_{y}-hydrates,
wherein n is 12, y is an integer from 1 to 24, C is CaO, and A is Al₂O₃.

3. The functionalized ceramic body of Claim 1 wherein an antibiotic is the biologically active moiety attached to the calcium aluminate containing phase.

4. The functionalized ceramic body of Claim 1 further comprising at least one of a retarder, water, an accelerator, a surfactant, a foaming agent, a fiber, a source of phosphate, an orthophosphate, and a reactive alumina, and combinations thereof.

5. The functionalized ceramic body of Claim 1 functionalized with the organic acid molecule and subsequently reacted with 3-maleimidopropionic acid N-hydroxysuccinimide ester for binding one or more peptides to said amino-functionalized calcium aluminate containing phase.

6. The functionalized ceramic body of Claim 1 that is an artificial prosthesis wherein said artificial prosthesis is selected from the group consisting of an artificial bone, artificial joint, in-vitro support structure, an in-vivo support structure, and a scaffolding matrix for support of cell, tissue, organ, and nerve growth.

7. A functionalized composition comprising:
a calcium aluminate containing phase that is functionalized with a linker group comprising an organic acid molecule for providing a reactive location for the attachment of another chemical moiety or a biologically active moiety to said calcium aluminate containing phase,
wherein said organic acid molecule has a formula of (CO₂H)ₐCₙH_{2n/2n-2} X_{d},
wherein X is selected from the group of CH₃, COOH, DCC-coupled COOH, OH, NH₂, a phosphate moiety and Br, wherein a is a number from 1 to 3, wherein n is a number from 12 to 18, and wherein d is a number from 1 to 3,
said reactive location being subsequently reacted with said other chemical moiety or said biologically active moiety to attach the other chemical entity or the biologically active moiety to said functionalized calcium aluminate containing phase.

8. The functionalized composition of Claim 7 wherein said calcium aluminate containing phase comprises one or more phases of calcium aluminate that result from the interaction of CₙA_{y} and CₙA_{y}-hydrates,
wherein n is 12, y is an integer from 1 to 24, C is CaO, and A is Al₂O₃.

9. The functionalized composition of Claim 7 wherein an antibiotic is the biologically active moiety attached to the calcium aluminate containing phase.

10. The functionalized composition of Claim 7 further comprising at least one of a retarder, water, an accelerator, a surfactant, a foaming agent, a fiber, a source of phosphate, an orthophosphate, and a reactive alumina, and combinations thereof.

11. The functionalized composition of Claim 7 functionalized with the organic acid molecule and subsequently reacted with 3-maleimidopropionic acid N-hydroxysuccinimide ester for binding one or more peptides to said amino-functionalized calcium aluminate containing phase.

12. A method of making an artificial prosthesis comprising:
mapping of a patient's identified bone or tissue structure;
creating a three dimensional pattern of said identified bone or tissue structure from said mapped bone or tissue structure;
creating a mold or negative of said identified bone or tissue structure from said pattern;
casting, into said mold thereby forming said artificial prosthesis, a functionalized composition comprising a calcium aluminate containing phase that is functionalized with a linker group comprising an organic acid molecule, wherein said organic acid molecule has a formula of (CO₂H)ₐCₙH_{2n/2n-2} X_{d},
wherein X is selected from the group of CH₃, COOH, DCC-coupled COOH, OH, NH₂, a phosphate group, and Br, wherein a is a number from 1 to 3, wherein n is a number from 12 to 18, and wherein d is a number from 1 to 3,
for providing a reactive location for the attachment of another chemical moiety or a biologically active moiety to said calcium aluminate containing phase,
said reactive location being subsequently reacted with said other chemical moiety or said biologically active moiety to attach the other chemical moiety or the biologically active moiety to said functionalized calcium aluminate containing phase.

13. The method of Claim 12 including wherein said calcium aluminate containing phase comprises one or more phases of calcium aluminate that result from the interaction of CₙA_{y} and CₙA_{y}-hydrates,
wherein n is 12, y is an integer from 1 to 24, C is CaO, and A is Al₂O₃.

14. The method of Claim 12 including wherein an antibiotic is the biologically active moiety attached to said calcium aluminate containing phase.

15. The method of Claim 12 including wherein said functionalized composition further comprises at least one of a retarder, water, an accelerator, a surfactant, a foaming agent, a fiber, a source of phosphate, an orthophosphate, and a reactive alumina, and combinations thereof.

16. The method of Claim 12 including wherein said calcium aluminate containing phase is functionalized with the organic acid molecule and subsequently reacted 3-maleimidopropionic acid N-hydroxysuccinimide ester for binding one or more peptides to said amino-functionalized calcium aluminate.

17. A functionalized ceramic body comprising an unfired hydratable composition comprising a calcium aluminate containing phase that is functionalized with a linker group comprising an organic acid molecule, wherein said organic acid molecule has a formula of (CO₂H)ₐCₙH_{2n/2n-2} X_{d}, wherein X is selected from the group of CH₃, COOH, DCC-coupled COOH, OH, a phosphate group, NH₂, and Br, wherein a is a number from 1 to 3, wherein n is a number from 12 to 18, and wherein d is a number from 1 to 3,
for providing a reactive location for the attachment of another chemical moiety or a biologically active moiety to the ceramic body,
said reactive location being subsequently reacted with said other chemical moiety or said biologically active moiety to attach the other chemical moiety or the biologically active moiety to said ceramic body.

18. The functionalized ceramic body of Claim 17 wherein the calcium aluminate containing phase comprises one or more phases of calcium aluminate that result from the interaction of CₙA_{y} and CₙA_{y}-hydrates,
wherein n is 12, y is an integer from 1 to 24, C is CaO, and A is Al₂O₃.

19. The functionalized ceramic body of Claim 17 wherein an antibiotic is the biologically active moiety attached to the calcium aluminate containing phase.

20. The functionalized ceramic body of Claim 17 further comprising at least one of a retarder, water, an accelerator, a surfactant, a foaming agent, a fiber, a source of phosphate, an orthophosphate, and a reactive alumina, and combinations thereof.

21. The functionalized ceramic body of Claim 17 functionalized with the organic acid molecule and subsequently reacted with 3-maleimidopropionic acid N-hydroxysuccinimide ester for binding one or more peptides to said amino-functionalized calcium aluminate containing phase.

22. The functionalized ceramic body of Claim 17 that is an artificial prosthesis wherein said artificial prosthesis is selected from the group consisting of an artificial bone, artificial joint, in-vitro support structure, an in-vivo support structure, and a scaffolding matrix for support of cell, tissue, organ, and nerve growth.

## Patentansprüche

1. Funktionalisierter Keramikkörper, der Folgendes umfasst:
eine Calciumaluminat enthaltende Phase, die mit einer Linker-Gruppe funktionalisiert ist, die ein organisches Säuremolekül umfasst, um eine reaktive Stelle für die Anbringung einer anderen chemischen Einheit oder einer biologisch aktiven Einheit an den Keramikkörper bereitzustellen,
wobei das organische Säuremolekül eine Formel von (CO₂H)ₐCₙH_{2n/2n-2}X_{d} aufweist,
wobei X aus der Gruppe von CH₃, COOH, N,N-Dicyclohexylcarbodiimid (DCC)-gekuppeltem COOH, OH, NH₂, einer Phosphateinheit und Br ausgewählt ist, wobei a eine Zahl von 1 bis 3 ist, wobei n eine Zahl von 12 bis 18 ist und wobei d eine Zahl von 1 bis 3 ist,
wobei die reaktive Stelle anschließend mit der anderen chemischen Einheit oder der biologisch aktiven Einheit zur Reaktion gebracht wird, um die andere chemische Einheit oder die biologisch aktive Einheit an die funktionalisierte, Calciumaluminat enthaltende Phase anzubringen.

2. Funktionalisierter Keramikkörper nach Anspruch 1, wobei die Calciumaluminat enthaltende Phase eine oder mehrere Phasen von Calciumaluminat umfasst, die aus der Wechselwirkung von CₙA_{y} und CₙA_{y}-Hydraten entstehen,
wobei n 12 ist, y eine ganze Zahl von 1 bis 24 ist, C CaO ist und A Al₂O₃ ist.

3. Funktionalisierter Keramikkörper nach Anspruch 1, wobei ein Antibiotikum die biologisch aktive Einheit ist, die an die Calciumaluminat enthaltende Phase angebracht wird.

4. Funktionalisierter Keramikkörper nach Anspruch 1, der weiter mindestens eines von Folgenden umfasst: einen Verzögerer, Wasser, einen Beschleuniger, ein Tensid, ein Schaummittel, eine Faser, eine Quelle für Phosphat, ein Orthophosphat und ein reaktives Aluminiumoxid und Kombinationen davon.

5. Funktionalisierter Keramikkörper nach Anspruch 1, der mit dem organischen Säuremolekül funktionalisiert ist und anschließend mit 3-Maleimidopropionsäure-N-hydroxysuccinimidester für die Bindung von einem oder mehreren Peptiden an die Amino-funktionalisiertes Calciumaluminat enthaltende Phase zur Reaktion gebracht wird.

6. Funktionalisierter Keramikkörper nach Anspruch 1, der eine künstliche Prothese ist, wobei die künstliche Prothese aus der Gruppe ausgewählt ist, die aus Folgenden besteht: einem künstlichen Knochen, künstlichem Gelenk, in-vitro-Stützstruktur, einer in-vivo-Stützstruktur und einer Gerüstmatrix für die Unterstützung von Zell-, Gewebe-, Organ- und Nervenwachstum.

7. Funktionalisierte Zusammensetzung, die Folgendes umfasst:
eine Calciumaluminat enthaltende Phase, die mit einer Linker-Gruppe funktionalisiert ist, die ein organisches Säuremolekül umfasst, um eine reaktive Stelle für die Anbringung einer anderen chemischen Einheit oder einer biologisch aktiven Einheit an die Calciumaluminat enthaltende Phase bereitzustellen,
wobei das organische Säuremolekül eine Formel von (CO₂H)ₐCₙH_{2n/2n-2}X_{d} aufweist,
wobei X aus der Gruppe von CH₃, COOH, DCC-gekuppeltem COOH, OH, NH₂, einer Phosphateinheit und Br ausgewählt ist, wobei a eine Zahl von 1 bis 3 ist, wobei n eine Zahl von 12 bis 18 ist und wobei d eine Zahl von 1 bis 3 ist,
wobei die reaktive Stelle anschließend mit der anderen chemischen Einheit oder der biologisch aktiven Einheit zur Reaktion gebracht wird, um die andere chemische Einheit oder die biologisch aktive Einheit an die funktionalisierte, Calciumaluminat enthaltende Phase anzubringen.

8. Funktionalisierte Zusammensetzung nach Anspruch 7, wobei die Calciumaluminat enthaltende Phase eine oder mehrere Phasen von Calciumaluminat umfasst, die aus der Wechselwirkung von CₙA_{y} und CₙA_{y}-Hydraten entstehen,
wobei n 12 ist, y eine ganze Zahl von 1 bis 24 ist, C CaO ist und A Al₂O₃ ist.

9. Funktionalisierte Zusammensetzung nach Anspruch 7, wobei ein Antibiotikum die biologisch aktive Einheit ist, die an der Calciumaluminat enthaltenden Phase angebracht wird.

10. Funktionalisierte Zusammensetzung nach Anspruch 7, die weiter mindestens eines von Folgenden umfasst: einen Verzögerer, Wasser, einen Beschleuniger, ein Tensid, ein Schaummittel, eine Faser, eine Quelle für Phosphat, ein Orthophosphat und ein reaktives Aluminiumoxid und Kombinationen davon.

11. Funktionalisierte Zusammensetzung nach Anspruch 7, die mit dem organischen Säuremolekül funktionalisiert ist und anschließend mit 3-Maleimidopropionsäure-N-hydroxysuccinimidester für die Bindung von einem oder mehreren Peptiden an die Amino-funktionalisiertes Calciumaluminat enthaltende Phase zur Reaktion gebracht wird.

12. Verfahren zur Herstellung einer künstlichen Prothese, das Folgendes umfasst:
Mapping einer identifizierten Knochen- oder Gewebestruktur eines Patienten;
Erzeugen eines dreidimensionalen Musters der identifizierten Knochen- oder Gewebestruktur aus der gemappten Knochen- oder Gewebestruktur;
Erzeugen einer Form oder eines Negativs der identifizierten Knochen- oder Gewebestruktur aus dem Muster;
Gießen in die Form, wodurch die künstliche Prothese ausgebildet wird, einer funktionalisierten Zusammensetzung, die eine Calciumaluminat enthaltende Phase umfasst, die mit einer Linker-Gruppe funktionalisiert ist, die ein organisches Säuremolekül umfasst,
wobei das organische Säuremolekül eine Formel von (CO₂H)ₐCₙH_{2n/2n-2}X_{d} aufweist,
wobei X aus der Gruppe von CH₃, COOH, DCC-gekuppeltem COOH, OH, NH₂, einer Phosphatgruppe und Br ausgewählt ist, wobei a eine Zahl von 1 bis 3 ist, wobei n eine Zahl von 12 bis 18 ist und wobei d eine Zahl von 1 bis 3 ist,
um eine reaktive Stelle für die Anbringung einer anderen chemischen Einheit oder einer biologisch aktiven Einheit an die Calciumaluminat enthaltende Phase bereitzustellen,
wobei die reaktive Stelle anschließend mit der anderen chemischen Einheit oder der biologisch aktiven Einheit zur Reaktion gebracht wird, um die andere chemische Einheit oder die biologisch aktive Einheit an die funktionalisierte, Calciumaluminat enthaltende Phase anzubringen.

13. Verfahren nach Anspruch 12, das einschließt, wobei die Calciumaluminat enthaltende Phase eine oder mehrere Phasen von Calciumaluminat umfasst, die aus der Wechselwirkung von CₙA_{y} und CₙA_{y}-Hydraten entstehen,
wobei n 12 ist, y eine ganze Zahl von 1 bis 24 ist, C CaO ist und A Al₂O₃ ist.

14. Verfahren nach Anspruch 12, das einschließt, wobei ein Antibiotikum die biologisch aktive Einheit ist, die an die Calciumaluminat enthaltende Phase angebracht wird.

15. Verfahren nach Anspruch 12, das einschließt, wobei die funktionalisierte Zusammensetzung weiter mindestens eines von Folgenden umfasst: einen Verzögerer, Wasser, einen Beschleuniger, ein Tensid, ein Schaummittel, eine Faser, eine Quelle für Phosphat, ein Orthophosphat und ein reaktives Aluminiumoxid und Kombinationen davon.

16. Verfahren nach Anspruch 12, das einschließt, wobei die Calciumaluminat enthaltende Phase mit dem organischen Säuremolekül funktionalisiert ist und anschließend mit 3-Maleimidopropionsäure-N-hydroxysuccinimidester für die Bindung von einem oder mehreren Peptiden an das Amino-funktionalisierte Calciumaluminat zur Reaktion gebracht wird.

17. Funktionalisierter Keramikkörper, der eine nicht gebrannte, hydratisierbare Zusammensetzung umfasst, die eine Calciumaluminat enthaltende Phase umfasst, die mit einer Linker-Gruppe funktionalisiert ist, die ein organisches Säuremolekül umfasst,
wobei das organische Säuremolekül eine Formel von (CO₂H)ₐCₙH_{2n/2n-2}X_{d} aufweist,
wobei X aus der Gruppe von CH₃, COOH, DCC-gekuppeltem COOH, OH, einer Phosphatgruppe, NH₂ und Br ausgewählt ist, wobei a eine Zahl von 1 bis 3 ist, wobei n eine Zahl von 12 bis 18 ist und wobei d eine Zahl von 1 bis 3 ist,
um eine reaktive Stelle für die Anbringung einer anderen chemischen Einheit oder einer biologisch aktiven Einheit an den Keramikkörper bereitzustellen,
wobei die reaktive Stelle anschließend mit der anderen chemischen Einheit oder der biologisch aktiven Einheit zur Reaktion gebracht wird, um die andere chemische Einheit oder die biologisch aktive Einheit an den Keramikkörper anzubringen.

18. Funktionalisierter Keramikkörper nach Anspruch 17, wobei die Calciumaluminat enthaltende Phase eine oder mehrere Phasen von Calciumaluminat umfasst, die aus der Wechselwirkung von CₙA_{y} und CₙA_{y}-Hydraten entstehen,
wobei n 12 ist, y eine ganze Zahl von 1 bis 24 ist, C CaO ist und A Al₂O₃ ist.

19. Funktionalisierter Keramikkörper nach Anspruch 17, wobei ein Antibiotikum die biologisch aktive Einheit ist, die an der Calciumaluminat enthaltenden Phase angebracht wird.

20. Funktionalisierter Keramikkörper nach Anspruch 17, der weiter mindestens eines von Folgenden umfasst: einen Verzögerer, Wasser, einen Beschleuniger, ein Tensid, ein Schaummittel, eine Faser, eine Quelle für Phosphat, ein Orthophosphat und ein reaktives Aluminiumoxid und Kombinationen davon.

21. Funktionalisierter Keramikkörper nach Anspruch 17, der mit dem organischen Säuremolekül funktionalisiert ist und anschließend mit 3-Maleimidopropionsäure-N-hydroxysuccinimidester für die Bindung von einem oder mehreren Peptiden an die Amino-funktionalisiertes Calciumaluminat enthaltende Phase zur Reaktion gebracht wird.

22. Funktionalisierter Keramikkörper nach Anspruch 17, der eine künstliche Prothese ist, wobei die künstliche Prothese aus der Gruppe ausgewählt ist, die aus Folgenden besteht: einem künstlichen Knochen, künstlichem Gelenk, in-vitro-Stützstruktur, einer in-vivo-Stützstruktur und einer Gerüstmatrix für die Unterstützung von Zell-, Gewebe-, Organ- und Nervenwachstum.

## Revendications

1. Corps céramique fonctionnalisé, comprenant :
une phase contenant de l'aluminate de calcium qui est fonctionnalisée par un groupement de liaison comprenant une molécule d'acide organique destinée à apporter un emplacement réactif pour la fixation d'un autre motif chimique ou d'un motif biologiquement actif au corps céramique,
où ladite molécule d'acide organique possède une formule (CO₂H)ₐCₙH_{2n/2n-2}X_{d},
où X est choisi dans le groupe constitué par CH₃, COOH, COOH couplé à du N,N-dicyclohexylcarbodiimide (DCC), OH, NH₂, un motif phosphate et Br, où a est un nombre allant de 1 à 3, où n est un nombre allant de 12 à 18, et où d est un nombre allant de 1 à 3,
ledit emplacement réactif étant ultérieurement réagi avec ledit autre motif chimique ou ledit motif biologiquement actif afin de fixer ledit autre motif chimique ou ledit motif biologiquement actif à ladite phase contenant de l'aluminate de calcium fonctionnalisée.

2. Corps céramique fonctionnalisé selon la revendication 1, dans lequel la phase contenant de l'aluminate de calcium comprend une ou plusieurs phases d'aluminate de calcium qui résultent de l'interaction de CₙA_{y} et de CₙA_{y}-hydrates,
où n vaut 12, y est un nombre entier allant de 1 à 24, C est CaO, et A est Al₂O₃.

3. Corps céramique fonctionnalisé selon la revendication 1, dans lequel un antibiotique constitue le motif biologiquement actif fixé à la phase contenant de l'aluminate de calcium.

4. Corps céramique fonctionnalisé selon la revendication 1, comprenant en outre au moins l'un parmi un retardateur, de l'eau, un accélérateur, un agent tensioactif, un agent moussant, une fibre, une source de phosphate, un orthophosphate, et une alumine réactive, et des combinaisons de ceux-ci.

5. Corps céramique fonctionnalisé selon la revendication 1, fonctionnalisé avec la molécule d'acide organique et ultérieurement réagi avec un ester N-hydroxysuccinimide d'acide 3-maléimidopropionique pour la fixation d'un ou plusieurs peptides à ladite phase contenant de l'aluminate de calcium à fonctionnalisation amino.

6. Corps céramique fonctionnalisé selon la revendication 1, qui est une prothèse artificielle, où ladite prothèse artificielle est choisie dans le groupe constitué par un os artificiel, une articulation artificielle, une structure de support *in vitro,* une structure de support *in vivo,* et une matrice d'échafaudage pour le support de la croissance de cellules, de tissus, d'organes, et nerveuse.

7. Composition fonctionnalisée, comprenant :
une phase contenant de l'aluminate de calcium qui est fonctionnalisée par un groupement de liaison comprenant une molécule d'acide organique destinée à apporter un emplacement réactif pour la fixation d'un autre motif chimique ou d'un motif biologiquement actif à ladite phase contenant de l'aluminate de calcium,
où ladite molécule d'acide organique possède une formule (CO₂H)ₐCₙH_{2n/2n-2}X_{d},
où X est choisi dans le groupe constitué par CH₃, COOH, COOH couplé à du DCC, OH, NH₂, un motif phosphate et Br, où a est un nombre allant de 1 à 3, où n est un nombre allant de 12 à 18, et où d est un nombre allant de 1 à 3,
ledit emplacement réactif étant ultérieurement réagi avec ledit autre motif chimique ou ledit motif biologiquement actif afin de fixer l'autre motif chimique ou le motif biologiquement actif à ladite phase contenant de l'aluminate de calcium fonctionnalisée.

8. Composition fonctionnalisée selon la revendication 7, dans laquelle ladite phase contenant de l'aluminate de calcium comprend une ou plusieurs phases d'aluminate de calcium qui résultent de l'interaction de CₙA_{y} et de CₙA_{y}-hydrates,
où n vaut 12, y est un nombre entier allant de 1 à 24, C est CaO, et A est Al₂O₃.

9. Composition fonctionnalisée selon la revendication 7, dans laquelle un antibiotique constitue le motif biologiquement actif fixé à la phase contenant de l'aluminate de calcium.

10. Composition fonctionnalisée selon la revendication 7, comprenant en outre au moins l'un parmi un retardateur, de l'eau, un accélérateur, un agent tensioactif, un agent moussant, une fibre, une source de phosphate, un orthophosphate, et une alumine réactive, et des combinaisons de ceux-ci.

11. Composition fonctionnalisée selon la revendication 7, fonctionnalisée avec la molécule d'acide organique et ultérieurement réagie avec un ester N-hydroxysuccinimide d'acide 3-maléimidopropionique pour la fixation d'un ou plusieurs peptides à ladite phase contenant de l'aluminate de calcium à fonctionnalisation amino.

12. Méthode de préparation d'une prothèse artificielle, comprenant :
la cartographie d'une structure osseuse ou tissulaire identifiée d'un patient ;
la création d'un modèle tridimensionnel de ladite structure osseuse ou tissulaire identifiée à partir de ladite structure osseuse ou tissulaire cartographiée ;
la création d'un moule ou d'un négatif de ladite structure osseuse ou tissulaire identifiée à partir dudit modèle ;
le moulage, dans ledit moule formant ainsi ladite prothèse artificielle, d'une composition fonctionnalisée comprenant une phase contenant de l'aluminate de calcium qui est fonctionnalisée par un groupement de liaison comprenant une molécule d'acide organique,
où ladite molécule d'acide organique possède une formule (CO₂H)ₐCₙH_{2n/2n-2}X_{d},
où X est choisi dans le groupe constitué par CH₃, COOH, COOH couplé à du DCC, OH, NH₂, un groupement phosphate et Br, où a est un nombre allant de 1 à 3, où n est un nombre allant de 12 à 18, et où d est un nombre allant de 1 à 3,
afin d'apporter un emplacement réactif pour la fixation d'un autre motif chimique ou d'un motif biologiquement actif à ladite phase contenant de l'aluminate de calcium, ledit emplacement réactif étant ultérieurement réagi avec ledit autre motif chimique ou ledit motif biologiquement actif afin de fixer l'autre motif chimique ou le motif biologiquement actif à ladite phase contenant de l'aluminate de calcium fonctionnalisée.

13. Méthode selon la revendication 12, dans laquelle ladite phase contenant de l'aluminate de calcium comprend une ou plusieurs phases d'aluminate de calcium qui résultent de l'interaction de CₙA_{y} et de CₙA_{y}-hydrates,
où n vaut 12, y est un nombre entier allant de 1 à 24, C est CaO, et A est Al₂O₃.

14. Méthode selon la revendication 12, dans laquelle un antibiotique constitue le motif biologiquement actif fixé à ladite phase contenant de l'aluminate de calcium.

15. Méthode selon la revendication 12, dans laquelle ladite composition fonctionnalisée comprend en outre au moins l'un parmi un retardateur, de l'eau, un accélérateur, un agent tensioactif, un agent moussant, une fibre, une source de phosphate, un orthophosphate, et une alumine réactive, et des combinaisons de ceux-ci.

16. Méthode selon la revendication 12, dans laquelle ladite phase contenant de l'aluminate de calcium est fonctionnalisée avec la molécule d'acide organique et ultérieurement réagie avec un ester N-hydroxysuccinimide d'acide 3-maléimidopropionique pour la fixation d'un ou plusieurs peptides audit aluminate de calcium à fonctionnalisation amino.

17. Corps céramique fonctionnalisé comprenant une composition hydratable non cuite comprenant une phase contenant de l'aluminate de calcium qui est fonctionnalisée par un groupement de liaison comprenant une molécule d'acide organique,
où ladite molécule d'acide organique possède une formule (CO₂H)ₐCₙH_{2n/2n-2}X_{d},
où X est choisi dans le groupe constitué par CH₃, COOH, COOH couplé à du DCC, OH, un groupement phosphate, NH₂, et Br, où a est un nombre allant de 1 à 3, où n est un nombre allant de 12 à 18, et où d est un nombre allant de 1 à 3,
afin d'apporter un emplacement réactif pour la fixation d'un autre motif chimique ou d'un motif biologiquement actif au corps céramique,
ledit emplacement réactif étant ultérieurement réagi avec ledit autre motif chimique ou ledit motif biologiquement actif afin de fixer l'autre motif chimique ou le motif biologiquement actif audit corps céramique.

18. Corps céramique fonctionnalisé selon la revendication 17, dans lequel la phase contenant de l'aluminate de calcium comprend une ou plusieurs phases d'aluminate de calcium qui résultent de l'interaction de CₙA_{y} et de CₙA_{y}-hydrates,
où n vaut 12, y est un nombre entier allant de 1 à 24, C est CaO, et A est Al₂O₃.

19. Corps céramique fonctionnalisé selon la revendication 17, dans lequel un antibiotique constitue le motif biologiquement actif fixé à la phase contenant de l'aluminate de calcium.

20. Corps céramique fonctionnalisé selon la revendication 17, comprenant en outre au moins l'un parmi un retardateur, de l'eau, un accélérateur, un agent tensioactif, un agent moussant, une fibre, une source de phosphate, un orthophosphate, et une alumine réactive, et des combinaisons de ceux-ci.

21. Corps céramique fonctionnalisé selon la revendication 17, fonctionnalisé avec la molécule d'acide organique et ultérieurement réagi avec un ester N-hydroxysuccinimide d'acide 3-maléimidopropionique pour la fixation d'un ou plusieurs peptides à ladite phase contenant de l'aluminate de calcium à fonctionnalisation amino.

22. Corps céramique fonctionnalisé selon la revendication 17, qui est une prothèse artificielle, où ladite prothèse artificielle est choisie dans le groupe constitué par un os artificiel, une articulation artificielle, une structure de support *in vitro,* une structure de support *in vivo,* et une matrice d'échafaudage pour le support de la croissance de cellules, de tissus, d'organes, et nerveuse.
